(19) Europäisches Patentamt / European Patent Office / Office européen des brevets

(11) **EP 4 257 676 A1**

(12) **EUROPEAN PATENT APPLICATION**
published in accordance with Art. 153(4) EPC

(43) Date of publication:
**11.10.2023 Bulletin 2023/41**

(21) Application number: **21885170.7**

(22) Date of filing: **26.10.2021**

(51) International Patent Classification (IPC):
*C12N 5/0783* (2010.01)     *C12N 5/10* (2006.01)
*C12N 15/12* (2006.01)     *C12N 15/13* (2006.01)
*A61P 31/12* (2006.01)     *A61P 35/00* (2006.01)

(86) International application number:
**PCT/CN2021/126480**

(87) International publication number:
**WO 2022/089443 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.10.2020 CN 202011159328**

(71) Applicant: **Chineo Medical Technology Co., Ltd.
Beijing 101111 (CN)**

(72) Inventor: **GU, Weiyue
Beijing 101111 (CN)**

(74) Representative: **Lavoix
Bayerstraße 83
80335 München (DE)**

Remarks:
The complete document including Reference
Table(s) and the Sequence Listing(s) can be
downloaded from the EPO website

(54) **MODIFIED IMMUNE CELL AND USE THEREOF**

(57)     A modified immune cell and a use thereof in immunotherapy, the immune cell being a PD-1$^+$T cell from peripheral blood. The immunotherapy is used in cancer treatment or treatment and prevention of diseases related to viral infections.

Figure 6 (continued)

**Description**

**TECHNICAL FIELD**

The invention belongs to the field of biomedical technologies, in particular to the field of immune cell therapies.

**BACKGROUND ART**

T cells belong to one type of the most important specific immune cells in the human body, and they have played an important role in the field of cell therapies in recent years. For example, autologous T cells can be modified and re-infused into patients for the cell therapies. However, given the heterogeneity of tumors, the individuality of the patient's MHC, and the randomness of the T cell receptor (TCR) production process (VDJ rearrangement), the T cell population that specifically recognizes harmful cells varies from patient to patient, in other words, the T cell population is one that comprises highly individualized TCR cohorts. How to quickly obtain a T cell population capable of specifically recognizing harmful cells (such as cancer cells or viral host cells) from the patient, and modify the T cells on this basis, and then apply them to cell therapies more efficiently and safely is a technical difficult problem.

**SUMMARY OF THE INVENTION**

The inventor of the invention proposes that PD-1-positive T cells (PD-1$^+$T cells) are a group of cells in a suppressive state, which are likely to be T cells that are capable of recognizing target cells and have undergone interaction with PD-L1 of target cells. When there is no history of autoimmune diseases in an individual, PD-1$^+$T cells can be considered a set of T cells that have attacked harmful cells. Because the T cells in this set have been in contact with harmful cells, it is believed that these T cells possess the ability to target harmful cells. The processed PD-1$^+$T cells in the peripheral blood are expected to be used for the treatment of various diseases, especially tumors and infectious diseases. The advantage of PD-1$^+$T cells is that they have a better characteristic of recognizing harmful cells, while the disadvantage is that the high expression of PD-1 makes these cells in a state of depletion, so that the function is inhibited and these cells are difficult to exert a killing effect. In order to make PD-1$^+$T cells have both a recognition characteristic and an immune activity, the invention proposes several modification methods to overcome the suppressive state of PD-1$^+$T cells, thereby completing the invention.

Thus, in a first aspect, the present application relates to a modified immune cell, wherein the immune cell is a PD-1-positive T (PD-1$^+$T) cell derived from peripheral blood. Preferably, the PD-1$^+$T cell is derived from peripheral blood mononuclear cells (PBMCs). For example, the PD-1$^+$T cell is a cell obtained by sorting PBMCs, for example by using PD-1 and CD3 as markers for the sorting.

In a preferred embodiment, the modification is a modification capable of attenuating or abolishing a suppressive state of the PD-1$^+$T cell. For example, the modification may be performed by genetic engineering and/or protein modification on the immune cell.

In a specific embodiment, the modification is a genetic modification of a PD1$^+$T cell, which results in loss or reduction of PD-1 expression in the PD1$^+$T cell. The genetic modification may be performed *in vivo* or *in vitro*. In one embodiment, the genetic modification is performed *in vivo* by gene editing or a gene therapy. In another embodiment, the genetic modification is performed *in vitro*, e.g., during preparation of the PD-1$^+$T cell. The genetic modification may be a knock-out or knock-down of endogenous PD-1 of the PD-1$^+$T cell. The means for knocking out the endogenous PD-1 gene include, but are not limited to, the CRISPR/Cas method, TALEN, etc.

In a specific embodiment, the modification is a modification of PD-1 on the surface of the PD-1$^+$T cell, so that the binding capacity of the PD-1 on the surface of the PD-1$^+$T cell to its ligand is reduced relative to that before the modification. In a specific embodiment, the modification is accomplished by mixing a PD-1 antibody with the PD-1$^+$T cell so that the PD-1 antibody competitively binds to PD-1 on the surface of the PD-1$^+$T cell, thereby reducing the binding capacity of PD-1 to its ligand. In one embodiment, the modification is performed *in vivo* by administration in combination with a PD-1 antibody. In another embodiment, the modification is performed *in vitro*, such as by adding a PD-1 antibody during preparation of the PD-1$^+$T cell. The PD-1 antibody is preferably a PD-1 monoclonal antibody or a single chain antibody.

In a further embodiment, the modified PD-1$^+$T cell has an enhanced receptor (ER) comprising: an extracellular domain (ECD) and an intracellular domain (ICD), wherein the ICD comprises a costimulatory molecule that elicits an immune cell activation signal, and the ECD comprises a portion that specifically binds to a target cell of the immune cell. In a specific embodiment, the portion that specifically binds to the target cell of the immune cell is selected from: a receptor, a ligand and an antibody of a membrane protein of the target cell; or a portion or fragment thereof having the function of binding to the target cell. Preferably, the ECD comprises a partial sequence of PD1, or an anti-PD-L1 antibody, preferably an anti-PD-L1scFv; and/or the ICD is derived from CD28.

Preferably, the modified immune cell has the ability of targeting to a target cell in a subject. In some embodiments, the target cell is a tumor cell, particularly a cancer cell. In some embodiments, the target cell is a host cell infected with a virus, such as hepatitis virus, preferably hepatitis B virus, hepatitis C virus, hepatitis D virus, or human papilloma virus (HPV). In some embodiments, the target cell of the modified immune cell is selected from one or more of a group of: tumor cells, cancer cells, and virus-infected cells.

In a further embodiment, the modified PD-1+T cell expresses a chimeric antigen receptor (CAR), wherein the CAR specifically recognizes another antigen which is different from the antigen recognized by the natural TCR of the immune cell. Preferably, the another antigen is CD19.

In a further embodiment, the modified PD-1+T cell comprises other modifications, such as a suicide switch.

In a second aspect, the present application provides a cell population comprising the modified immune cell of the first aspect.

In a third aspect, the present application provides a method for preparing modified immune cells, the method comprising: (a) sorting PD-1+T cells from peripheral blood; and (b) performing one or more of the following treatments on the PD-1+T cells sorted in step (a): i. knocking out or knocking down the expression of PD-1; ii. mixing with a PD-1 antibody; iii. allowing the cell to express an enhanced receptor (ER); iv. allowing the cell to express a chimeric antigen receptor (CAR); and v. allowing the cell to express a modification that controls cell death, such as a suicide switch. In a preferred embodiment, the treatments of step (b) at least comprise allowing the cell to express the enhanced receptor (ER). Further, the treatments of step (b) comprise allowing the cell to express a chimeric antigen receptor (CAR). In another embodiment, or in embodiments on this basis, the treatments of step (b) comprise knocking out or knocking down the expression of PD-1 and/or mixing with a PD-1 antibody. Additionally, the treatments of step (b) comprises allowing the cell to express a suicide switch. The treatments may be carried out *in vivo* or *in vitro*. The treatments may be performed before, after, or simultaneously with the reinfusion of the modified immune cells to the subject. In a specific embodiment, the sorting of step (a) of the method comprises performing PD-1+ cell sorting and CD3+ cell sorting from peripheral blood mononuclear cells in either order. In a preferred embodiment, the treatments at least comprise i. knocking out or knocking down the expression of PD-1.

In a fourth aspect, the present application relates to a modified immune cell prepared by the method of the third aspect.

In a fifth aspect, the present application relates to a pharmaceutical composition comprising the modified immune cell of the first or fourth aspect, or the cell population of the second aspect.

In a sixth aspect, the present application relates to the use of the immune cell of the first or fourth aspect or the cell population of the second aspect in a therapy, or in the manufacture of a medicament. The therapy or the medicament is used for (1) treatment of tumors, and/or (2) treatment or prevention of diseases or symptoms related to viral infections, or prevention of recurrence of diseases or symptoms related to viral infections. Preferably, the viral infections are chronic viral infections. In a specific embodiment, the virus is hepatitis virus, preferably hepatitis B virus. In another specific embodiment, the virus is human papilloma virus. The immune cell of the invention can be used to eliminate residual viral components after treatment in a subject, and prevent the recurrence of viral infections.

**BRIEF DESCRIPTION OF THE DRAWINGS**

Figure 1 is a photograph showing the tumor-bearing condition of a PDX mouse in Example 1.

Figure 2 is a photograph showing a tumor-bearing mouse dissected for isolating tumor tissues in Example 1.

Figure 3A-B shows the results of the secretion of IFNγ as detected by ELISPOT experiment, using PD-1+T cells or PD-1-T cells from peripheral blood of a patient which were co-cultured with tumor tissues of the patient, the results showing the difference in recognition and responsiveness of the two T cells for the tumor; (A) ELISPOT staining photographs and ELISPOT statistical results in the case of co-culturing for 24 hours; and (B) ELISPOT staining photographs and ELISPOT statistical results in the case of co-culturing for 48 hours.

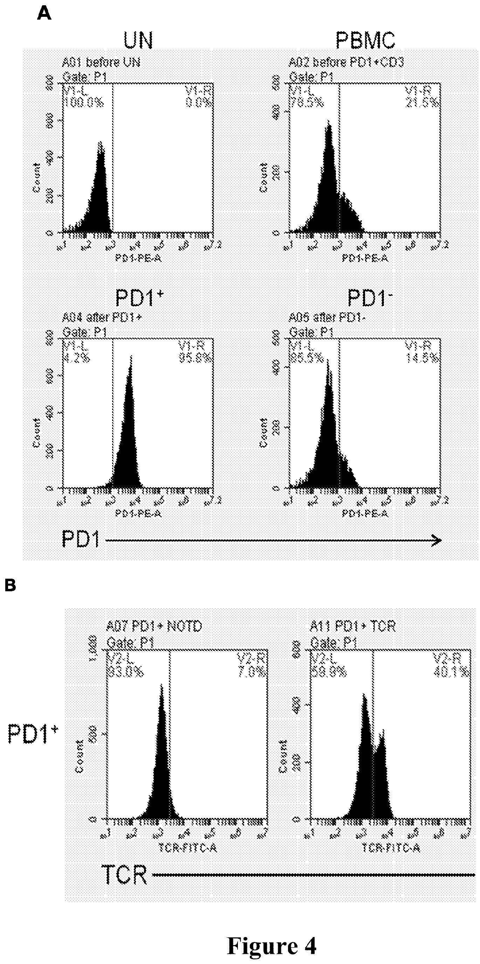

Figure 4A-E show the results of an *in vitro* experiment detecting the effect of PD-1 knock-out on PD-1+T cell function. (A) the PD-1 sorting efficiency detected by flow cytometry; (B) the TCR expression detected by flow cytometry, with cells not transfected with TCR-expressing virus as control (PD-1+ NOTD); (C) the PD-1 knock-out effect detected by flow cytometry in PD-1+T cells expressing and not expressing TCR, with untreated cells and cells subjected to empty electric shock as controls for both types of cells; and (D-E) the secretion of IFNγ (D) and IL-2 (E) by T cells as detected by ELISA experiment after T cells were co-cultured with J82-NY tumor cells.

Figure 5A-D show the results of an *in vitro* experiment detecting the effect of the enhanced receptor on PD-1+T cell function. (a) the PD-1 sorting efficiency detected by flow cytometry; (B-C) the secretion of IFN γ (B) and IL-2 (C) by T cells as detected by ELISA experiment after T cells expressing and not expressing enhanced receptors were co-cultured with J82-NY tumor cells; and (D) the secretion of IFN γ by T cells expressing and not expressing enhanced receptors in 96-well plates coated with OKT3 and/or PD-L1 proteins, as detected by ELISA experiment.

Figures 6A-F show the results of an *in vivo* experiment in mice seeded with J82-NY-ESO1 tumor cells in Example 4, demonstrating the effect of enhanced receptors on the tumor-inhibiting function of PD-1$^+$T cells, (a) PBS control; (B) PD-1$^+$NOTD; (C) PD-1$^-$TCR-T cells; (D) PD-1$^+$TCR-T cells; (E) PD-1$^+$-V 1E-TCR-T cells; and (F) a summary chart of average values among each group.

Figures 7A-C show the results of an *in vivo* experiment in Example 4 in mice seeded with tumor tissues from a patient with colon cancer, demonstrating the effect of enhanced receptors on the tumor-inhibiting function of PD-1$^+$T cells, (a) PBS control; (B) PD-1-T cells; (C) PD-1$^+$T cells; and (D) PD-1$^+$-V2E-T cells.

Figure 8 shows the liver function- and HBV-related indices in patients (A-B) and a control subject (C) in Example 6.

Figure 9 shows the biological process of HBV in host hepatocytes.

Figure 10 shows cytotoxic T lymphocyte (CTL) attack and target cell immune escape.

Figure 11 shows that ScTIL eliminates residual components (excluding active viruses) of HBV in liver cells.

Figure 12 is a plasmid map of the lentiviral vector construct pLenti-TCR-NY ESO1 for introducing the TCR for the recognition NY-ESO-1.

Figure 13 is a plasmid map of the lentiviral vector construct pLenti-V1E-T2A-TCR for introducing the enhanced receptor V1E.

Figure 14 is a plasmid map of the lentiviral vector construct pLenti-V2E-T2A-TCR for introducing the enhanced receptor V2E.

Figure 15 is a structural diagram of the CD19 CAR used as an expansion factor.

Figure 16 is the results of imaging examination for patients 1-4 before and after treatment in Example 5.

## DETAILED DESCRIPTION OF EMBODIMENTS

### Definitions

The practice of some methods disclosed herein employ, unless otherwise indicated, conventional techniques of immunology, biochemistry, chemistry, molecular biology, microbiology, cell biology, genomics and recombinant DNA, which are within the skill of the art.

The term "about" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated, the term "about" meaning within an acceptable error range for the particular value should be assumed.

Herein, the term "gene" refers to a nucleic acid (e.g., a DNA, such as a genomic DNA and cDNA) and its corresponding nucleotide sequence that encodes an RNA transcript. A genomic DNA herein includes intervening, non-coding regions as well as regulatory regions, and can include 5' and 3' ends. In some uses, the term encompasses the transcribed sequences, including 5' and 3' untranslated regions (5'-UTR and 3'-UTR), exons and introns. In some genes, a transcribed region will contain "open reading frames" that encode polypeptides. In some uses of the term, a "gene" comprises only the coding sequence necessary for encoding a polypeptide, e.g., an "open reading frame" or "coding region". In some cases, a gene does not encode a polypeptide, for example, a ribosomal RNA gene (rRNA) and a transfer RNA (tRNA) gene. In some cases, the term "gene" includes not only the transcribed sequences, but also includes non-transcribed regions, including upstream and downstream regulatory regions, enhancers and promoters. A gene can refer to an "endogenous gene" or a native gene in its natural location in the genome of an organism. A gene can refer to an "exogenous gene" or a non-native gene. A non-native gene can refer to a gene not normally found in the host organism but which is introduced into the host organism by gene transfer. A non-native gene can also refer to a gene not in its natural location in the genome of an organism. A non-native gene can also refer to a naturally occurring nucleic acid or polypeptide sequence that comprises mutations, insertions and/or deletions (e.g., non-native sequences).

The term "nucleotide" generally refers to a base-sugar-phosphate combination. A nucleotide can include a nucleotide analog or derivative and a synthetic nucleotide. For the purpose of detection, a nucleotide can be labeled by known techniques. Detectable labels can include, for example, radioactive isotopes, fluorescent labels, chemiluminescent labels, bioluminescent labels and enzyme labels.

The terms "polynucleotide", "oligonucleotide", and "nucleic acid" are used interchangeably to refer to a polymeric form of nucleotides of any length, either deoxyribonucleotides or ribonucleotides, or analogs thereof, either in single-, double- or multi-stranded form. A polynucleotide can be exogenous or endogenous to a cell. A polynucleotide can be a gene or a fragment thereof. A polynucleotide can be a DNA or an RNA. A polynucleotide can have any three dimensional structure, and can perform any function, known or unknown. A polynucleotide can comprise one or more analogs (e.g., altered backbones, sugars or nucleobases).

The term "expression" refers to one or more processes by which a polynucleotide is transcribed from a DNA template (such as into an mRNA or other RNA transcripts) and/or by which a transcribed mRNA is subsequently translated into peptides, polypeptides or proteins. Transcripts and encoded polypeptides can be collectively referred to as "gene products." If the polynucleotide is derived from a genomic DNA, expression can include splicing of the mRNA in a eukaryotic cell. "Up-regulated" or "down-regulated" expression generally refers to an increased or decreased expression level of a polynucleotide (e.g., an RNA such as an mRNA) and/or a polypeptide sequence relative to its expression level in a wild-type state.

The term "regulating" with reference to expression or activity refers to altering the level of expression or activity. Regulation can occur at the transcription level and/or translation level.

The terms "peptide", "polypeptide", and "protein" are used interchangeably herein to refer to a polymer of at least two amino acid residues joined by a peptide bond(s). The terms "peptide", "polypeptide", and "protein" are used interchangeably herein to refer to a polymer of at least two amino acid residues joined by a peptide bond(s). The term does not mean a polymer of a specific length, nor does it mean that the polymer is natural, modified or synthetic. In some cases, the polymers can be interrupted by a non-amino acid(s). The terms include amino acid chains of any length, including full length proteins, and proteins with or without secondary and/or tertiary structure (e.g., domains). The terms also encompass amino acid polymers that have been modified, for example, by disulfide bond formation, glycosylation, lipidation, acetylation, phosphorylation, oxidation, and any other manipulations such as conjugation with a labeling component. The term "amino acid" as used herein, generally refers to natural and non-natural amino acids, including but not limited to modified amino acids and amino acid analogs. The term "amino acid" includes both D-amino acids and L-amino acids.

The term "fusion" can refer to a protein and/or nucleic acid comprising one or more non-native sequences (e.g., moieties). A fusion can comprise one or more identical or different non-native sequences. A fusion can be a chimera. A fusion can comprise a nucleic acid affinity tag, a barcode and a peptide affinity tag. A fusion can provide a signal for locating the polypeptide at a subcellular site, e.g., a nuclear localization signal (NLS) for targeting to the nucleus, a mitochondrial localization signal for targeting to the mitochondria, a chloroplast localization signal for targeting to a chloroplast, an endoplasmic reticulum (ER) retention signal, etc. A fusion can provide a non-native sequence (e.g., affinity tag) that can be used for tracking or purification. A fusion can comprise a small molecule such as biotin or a dye such as Alexa fluor dyes, Cyanine3 dye and Cyanine5 dye.

The term "antigen", as used herein, refers to a molecule or fragment thereof capable of being bound by a selective binding agent. As an example, an antigen can be a ligand that can be bound by a selective binding agent such as a receptor. As another example, an antigen can be an antigenic molecule that can be bound by a selective binding agent such as an immunological protein (e.g., an antibody). An antigen can also refer to a molecule or fragment thereof capable of being used in an animal to produce antibodies capable of binding to that antigen.

The term "antibody", as used herein, refers to a proteinaceous binding molecule with immunoglobulin-like functions. The term antibody includes an antibodies (e.g., monoclonal and polyclonal antibodies), as well as derivatives, variants and fragments thereof. Antibodies include, but are not limited to, immunoglobulins (Ig's) of different classes (i.e., IgA, IgG, IgM, IgD and IgE) and subclasses (such as IgG1, IgG2, etc.). A derivative, variant or fragment thereof can refer to a functional derivative, variant or fragment which retains the binding specificity (e.g., complete and/or partial) of the corresponding antibody. Antigen-binding fragments include Fab, Fab', F(ab')2, variable fragments (Fvs), single chain variable fragments (scFvs), minibodies, diabodies, and single-domain antibodies ("sdAb" or "nanobodies" or "camelid antibodies"). The term antibody includes antibodies and antigen-binding fragments of antibodies that have been optimized, engineered or chemically conjugated. Examples of antibodies that have been optimized include affinity-matured antibodies. Examples of antibodies that have been engineered include Fc optimized antibodies (e.g., antibodies optimized in the fragment crystallizable region) and multispecific antibodies (e.g., bispecific antibodies).

The term "TCR" or "T cell receptor" has a meaning generally understood in the art. TCR is the basis of T cell-mediated antigen recognition and is a key point in the immune system. TCR is highly diverse, and the relationship between this diversity and diseases is a hot research topic in the field of immunology. The set of TCRs contained by T cells under certain conditions or at certain time points can be called a TCR repertoire or TCR profile, which may change greatly with the occurrence and development of diseases.

The terms "subject", "individual", and "patient" are used interchangeably herein to refer to a vertebrate, preferably a mammal such as a human. Mammals include, but are not limited to, murines, simians, humans, farm animals, sport animals and pets. Tissues, cells and their progenies of a biological entity obtained *in vivo* or cultured *in vitro* are also encompassed.

Herein, the term "treatment" refers to an approach for obtaining beneficial or desired results (including but not limited to a therapeutic benefit and/or a prophylactic benefit). For example, treatment can comprise administering the modified immune cell or the cell population comprising the immune cell of the invention. The therapeutic benefit refers to any therapeutically relevant improvement in one or more diseases or symptoms under treatment. For the prophylactic benefit, the modified immune cell of the invention can be administered to a subject at risk of developing a particular disease or symptom, or to a subject having one or more physiological signs of a disease, even though the disease or symptom may not have yet been manifested.

The term "effective amount" or "therapeutically effective amount" refers to the amount of a composition, for example a composition comprising the modified immune cell of the invention such as a lymphocyte (e.g., a T lymphocyte and/or NK cell), that is sufficient to result in a desired activity upon administering the composition at this amount to a subject in need thereof.

Immune cells

The immune cells of the invention are derived from peripheral blood, such as peripheral blood mononuclear cells (PBMCs), peripheral blood lymphocytes (PBLs) and other blood cell subsets, including but not limited to T cells, natural killer cells, monocytes, monocyte-precursor cells, hematopoietic stem cells or non-pluripotent stem cells. In some cases, the immune cell of the invention can be any immune cell, including any T cell such as tumor-infiltrating cells (TILs). The immune cells can be derived from a subject to be treated (e.g., a patient). The subject can be a mammal, such as a mouse, monkey or human. In a specific embodiment, the immune cells are isolated from peripheral blood mononuclear cells (PBMCs) of a subject, such as a human subject.

T cells can be obtained from blood collected from a subject using any technique, such as Ficoll separation. Cells from the circulating blood of a subject can be obtained by apheresis or leukapheresis. The apheresis product typically contains lymphocytes, including T cells, monocytes, granulocytes, B cells, and other nucleated white blood cells, and also contains red blood cells and platelets. The cells collected by apheresis can be washed to remove the plasma fraction, and the cells can be placed in a suitable buffer or medium, such as phosphate buffered saline (PBS), for subsequent processing steps. After washing, the cells can be resuspended in a variety of biocompatible buffers, such as Ca-free, Mg-free PBS. Alternatively, the undesired components of the apheresis sample can be removed and the cells are directly resuspended in culture media. Samples can be provided directly by the subject, or indirectly through one or more intermediaries, such as a sample collection service provider or a medical provider (e.g., a physician or nurse). In some embodiments, isolating T cells from peripheral blood leukocytes can comprise lysing the red blood cells and separating peripheral blood leukocytes from monocytes for example by PERCOL™ gradient centrifugation.

A specific subset of immune cells such as T cells, such as T cell subset comprising the immune cells of the invention positive for PD-1, can be further isolated by positive or negative selection techniques. Without being limited to any theory, it is believed that PD-1-positive cells are likely to represent a cell population that has experienced interaction with target cells; therefore, this cell population can recognize target cells. In a specific embodiment, the immune cells such as PBMCs obtained from peripheral blood are sorted with PD-1 as a marker. For example, PD-1$^+$T cells can be sorted using PD-1 antibodies by magnetic sorting (e.g., using magnetic beads) or flow cytometry, preferably using magnetic beads for sorting.

It is considered in the invention that the sorted PD-1-positive T cell subsets are T cell subsets that have interacted with harmful cells that are intended to be targeted; therefore, the T cells contained therein have a TCR repertoire or TCR profile that can specifically recognize antigens on harmful cells. In order to enable T lymphocytes used in a cell therapy to target and identify harmful cells, such as tumor cells, one of the practices in the prior art is obtaining TCRs that can target the harmful cells, such as tumor cells by screening a TCR library, obtaining their genetic information, and accordingly, genetically engineering T cells from patients to impart them the ability to recognize antigens on harmful cells. Unlike this technique, which imparts T cell recognition ability through exogenous modification, the invention can ensure the targeting ability and safety of the cells used for treatment without TCR screening or introducing exogenous TCRs into the T cells used for treatment, but by directly sorting those T cells, that have a high probability of having TCRs specific for harmful cells, from the patient's autologous cells. Thus, those skilled in the art will understand that the invention does not require T cells to have one or more specific TCRs, as the TCR repertoire can vary greatly depending on the patient and the disease state. The invention requires that there is a recognition between the T cell population and the harmful cells, and this recognition ability is mainly obtained by sorting PD-1-positive T cells in the invention. It is also for this reason that the methodology of the invention can be used to target a variety of harmful cells and treat a variety of diseases.

In the context of the invention, "harmful cells" refer to cells targeted and attacked by immune cells, especially tumor cells, cancer cells, and virus-infected cells.

The T cells of the invention may also be a CD3-positive, CD4-positive, CD4-negative or CD8-positive T cell subset. Any technique in the art can be used to sort cell subsets with or without specific markers. For example, CD3$^+$T cells can be sorted by a matrix (such as magnetic beads) coupled with antibodies to the relevant markers, such as CD3 magnetic beads or CD3/CD28 magnetic beads. Changing the cell concentration can contribute to maximum contact with the substrate surface, such as the surface of magnetic beads. In addition, negative selection of a cell population can be achieved, for example, with a combination of antibodies to surface markers specific for undesired cells. One suitable technique comprises cell sorting via negative magnetic immunoadherence, which utilizes a cocktail of monoclonal antibodies to cell surface markers present on the undesired cells. For example, to isolate CD4$^+$ cells, a monoclonal antibody cocktail can comprise antibodies to CD14, CD20, CD11b, CD16, HLA-DR and CD8.

In some embodiments, the immune cell is a cell in an enriched cell population. One or more desired cell types can be enriched by any suitable method, for example, by treating a cell population to trigger expansion and/or differentiation to a desired cell type, or by stopping the growth of an undesired cell type, or by killing or lysing an undesired cell type, or by purifying a desired cell type (e.g., by purifying on an affinity column to retain desired or undesired cell types on the basis of one or more cell surface markers).

Modifications

Due to interaction with target cells, PD-1$^+$T cells are exhausted and "passivated", and their potency and function are weakened. The efficacy of PD-1$^+$T cells is restored and improved by modifying them in the invention.

The PD-1 expression of the modified immune cells of the invention is knocked out or knocked down. Herein, the "knock-out" of gene expression refers to substantial elimination of the expression of the gene. Herein, the "knock-down" of gene expression refers to reduction of the expression of the gene. In a preferred embodiment, the endogenous PD-1 expression of the modified immune cells of the invention is knocked out. The knock-out or knock-down of PD-1 may be achieved using any method known in the art, including but not limited to: RNAi (including using siRNA or shRNA), CRISPR-Cas method, transcription activator-like effector nuclease (TALEN) technology, site-directed mutagenesis, zinc finger nuclease (ZFN) technology, or combinations thereof.

In a preferred embodiment, an endogenous PD-1 of the immune cell is knocked out using the CRISPR/Cas method, comprising using a guide RNA (gRNA) capable of hybridizing to the target gene PD-1 and a Cas protein or its coding nucleic acid molecule. In a specific embodiment, an sgRNA targeting to PD-1 is used to knock out the endogenous PD-1 of the immune cell, preferably the sgRNA has a nucleotide sequence set forth in SEQ ID NO: 4. In a specific embodiment, the Cas9 protein or its coding nucleic acid molecule is used to knock out PD-1. The Cas9 protein may be derived from *Streptococcus pyogenes, Streptococcus thermophilus* or other species. For example, Cas9 may include spCas9, Cpfl, CasY, CasX or saCas9.

In one aspect, the modified immune cell of the invention comprises an enhanced receptor (ER). The enhanced receptor can be used to provide further control over immune cell activities, such as but not limited to, immune cell activation and expansion. Binding of the enhanced receptor to its ligand can yield an immune cell activation signal rather than an immune cell inactivation signal in the modified immune cell. Eliciting the immune cell activation signal rather than the immune cell inactivation signal in the modified immune cell may minimize an immune-suppressive effect in the immune cell. Minimizing an immune-suppressive effect in the immune cell can increase the effectiveness of the immune cell in an immune response, for example by increasing immune cell cytotoxicity against a target cell, such as a tumor cell or infected cell.

The enhanced receptor can comprise an extracellular domain (ECD) of a protein. The protein can be a signaling receptor, or any functional fragment, derivative or variant thereof. In some cases, the signaling receptor can be a membrane-bound receptor. A signaling receptor can, in response to ligand binding, induce one or more signaling pathways in a cell. In some cases, the signaling receptor can be a non-membrane-bound receptor. The enhanced receptor can comprise a fragment (e.g., an extracellular domain) of a receptor selected from: a G-protein coupled receptor (GPCR), an integrin receptor, a cadherin receptor, a catalytic receptor (e.g., a kinase), a death receptor, a checkpoint receptor, a cytokine receptor, a chemokine receptor, a growth factor receptor, a hormone receptor, and an immune receptor.

In some embodiments, the enhanced receptor comprises a fragment of an immune checkpoint receptor, which may be involved in regulation of the immune system. Non-limiting examples of such receptors include, but are not limited to, PD-1, CTLA-4, BTLA, KIR, IDO, LAG3, TIM-3, TIGIT, SIRPα, and NKG2D, preferably PD-1.

The enhanced receptor can comprise a fragment binding to any suitable immune checkpoint receptor ligand. Non-limiting examples of such ligands include, but are not limited to, B7-1, B7-H3, B7-H4, HVEM (Herpesvirus Entry Mediator), AP2M1, CD80, CD86, SHP-2, PPP2R5A, MHC (e.g., class I and class II), CD47, CD70, PD-L1 (or PDL1) and PD-L2. The regions where the enhanced receptor bind to such ligands can be natural receptors for such ligands or monoclonal antibodies to such ligands.

The enhancer receptor also comprises an intracellular domain (ICD), which may be a costimulatory molecule or a fragment, variant, or derivative thereof that elicits an immune cell activation signal. In some embodiments, the costimulatory molecule can be used to regulate a proliferative and/or survival signal in the immune cell. In some embodiments, the ICD is an intracellular domain of a costimulatory molecule selected from an MHC class I protein, an MHC class II protein, a TNF receptor protein, an immunoglobulin-like protein, a cytokine receptor, an integrin, an SLAM protein, an activating NK cell receptor, BTLA or a Toll ligand receptor. In a preferred embodiment, the costimulatory molecule is a T cell costimulatory molecule, preferably a positive T cell costimulatory molecule, preferably CD28.

The ECD and the ICD of the enhanced receptor can be joined by a transmembrane domain. In some embodiments, the transmembrane domain comprises a polypeptide. The transmembrane polypeptide can have any suitable polypeptide sequence. In some cases, the transmembrane polypeptide comprises a polypeptide sequence of a transmembrane portion of an endogenous or wild-type transmembrane protein, or a variant of the polypeptide sequence with at least one amino acid change. In some embodiments, the transmembrane polypeptide comprises a non-natural polypeptide sequence, such as the sequence of a polypeptide linker. The polypeptide linker may be flexible or rigid. The polypeptide linker can be structured or unstructured. In some embodiments, the transmembrane polypeptide transmits a signal from the ECD to the ICD, for example a signal indicating ligand-binding. In some embodiments, the ECD comprises a transmembrane domain. In some embodiments, the ICD comprises a transmembrane domain.

Means of transfection with the gene of an enhanced receptor can be transfecting the PD-1$^+$T cells with the gene of the enhanced receptor by electrotransfection of the mRNA of the enhanced receptor into the PD-1$^+$T cells, or through lentiviral, adeno-associated viral, or non-viral vectors.

The enhanced receptor is a transmembrane protein expressed on the surface of an immune cell, which is composed of an extracellular domain (ECD) and an intracellular domain (ICD), wherein the ICD contains a costimulatory molecule eliciting an immune cell activation signal, which can elicit an immune cell activation signal, and the ECD can bind to a target cell of the immune cell, and is a receptor, ligand, and or antibody for a membrane protein of the target cell of the PD-1$^+$T cell, or is a complete sequence structure of any substance that is capable of binding to a target cell, or a portion thereof comprising the binding domain.

In a specific embodiment, the enhanced receptor is V1E or V2E, the ECD comprises a fragment of PD-1 (e.g., a fragment of an extracellular domain of PD-1) or an scFv sequence of an anti-PD-L1 monoclonal antibody, respectively, and the ICD of the enhanced receptor comprises an intracellular signaling domain of CD28. More preferably, the enhancer receptor comprises a transmembrane region of CD28 or a transmembrane region of CD8. In a most preferred embodiment, the enhanced receptor comprises or consists of a sequence selected from SEQ ID NO: 1 or SEQ ID NO: 2, or a sequence having at least 75%, 80%, 85%, 90%, 95%, 97% or 99% homology thereto.

In some embodiments, the modified immune cell of the invention comprises a chimeric antigen receptor (CAR). When the CAR is capable of recognizing a target cell, the enhanced receptor-modified immune cell, such as a T cell, has a stronger immune cell activation function than a cell not modified by the enhanced receptor. In some cases, the CAR can facilitate the modified immune cells to expand.

In a preferred embodiment, the CAR specifically recognizes another antigen which is different from the antigen recognized by the natural TCR of the immune cell. Preferably, the CAR comprises an antigen interacting domain capable of binding to a B cell surface protein (antigen binding domain), a transmembrane domain and an intracellular signaling domain. The B cell surface protein can be any protein that may be found on the surface of a B cell, preferably CD19. In some embodiments, the antigen interacting domain of the CAR can be capable of binding to a surface protein on a non-B cell, as long as the binding to the surface protein does not significantly compromise the general health status or the immune system of the host.

Non-limiting examples of the antigen binding domain include, but are not limited to, a monoclonal antibody, a polyclonal antibody, a recombinant antibody, a human antibody, a humanized antibody, a murine antibody, or a functional derivative, variant or fragment thereof, including, but not limited to, Fab, Fab', F(ab')2, Fv, a single-chain Fv (scFv), a minibody, a diabody, and a single-domain antibody such as a heavy chain variable domain (VH), a light chain variable domain (VL) and a variable domain (VHH) of camelid-derived nanobody. In some embodiments, the antigen binding domain comprises at least one of Fab, Fab', F(ab')2, Fv and scFv, preferably scFv. In some embodiments, the antigen binding domain comprises an antibody mimetic. The antibody mimetic refers to a molecule capable of binding to a target molecule with an affinity comparable to that of an antibody. In some embodiments, the antigen binding domain comprises a transmembrane receptor, or any derivative, variant or fragment thereof. For example, the antigen binding domain can comprise at least a ligand binding domain of the transmembrane receptor.

In some embodiments, the antigen interacting domain of the CAR is capable of binding to a surface protein or a fragment thereof on a B cell that is coupled (e.g., via covalent and/or non-covalent bonds) to a particle (e.g., a nanoparticle). The particles may be any particulate material comprising an organic and/or inorganic material. The particles may have various shapes and sizes. The particles may be about 1 nanometer (nm) to about 50 micometers (μm) in at least one dimension. The particles may be at least about 1 nm, 5 nm, 10 nm, 50 nm, 100 nm, 500 nm, 1 μm, 5 μm, 10 μm, 50 μm or larger in at least one dimension. The particles may be at most about 50 μm, 10 μm, 5 μm, 1 μm, 500 nm, 100 nm, 50 nm, 10 nm, 5 nm, 1 nm or less in at least one dimension. The particles may be nanoparticles, microparticles, nanospheres, micropsheres, nanorods, microrods, nanofibers, nanoribbons, etc. Examples of the particles include metal nanoparticles (e.g., gold nanoparticles, silver nanoparticles, and iron nanoparticles), intermetallic nanosemiconductor nanoparticles, core-shell nanoparticles, particles with an inorganic core with a polymer shell, particles with an organic core with a polymer shell, and mixtures thereof. Alternatively, the particles can be organic nanoparticles, such as crosslinked polymers, hydrogel polymers, biodegradable polymers, polylactide (PLA), polyglycolide (PGA), polycaprolactone (PCL), copolymers, polysaccharides, starch, cellulose, chitosan, polyhydroxyalkanoates (PHA), PHB, PHV, lipids, peptides, peptide amphiphiles, polypeptides (e.g., proteins), or combinations thereof. The particles presenting the B cell surface protein on the surface may be introduced *in vitro* to the immune cell comprising the CAR that binds the B cell surface protein. Alternatively or in addition, the particles presenting the B cell surface protein may be introduced *in vivo* (e.g., by local or systemic injection) along with the immune cell comprising the CAR. Such particles may be used to expand a population of immune cells comprising the CAR *in vitro* or *in vivo*.

In some embodiments, the antigen interacting domain of the CAR can be capable of binding to a B cell surface protein or a fragment thereof on a dead B cell. B cell apoptosis can occur before or after development of an immune response. Thus, a dead B cell or its debris can still have the B cell surface protein or fragment thereof presented on the surface. The ability of the CAR to target both live and dead B cells may increase the probability of the modified immune cell comprising the CAR to bind the B cell surface protein and initiate signaling of the intracellular signaling domain. In some cases, the signaling of the intracellular signaling domain may promote expansion (proliferation) of the immune cell comprising the CAR.

The intracellular signaling domain of a CAR can induce the activity of the modified immune cell. The intracellular signaling domain can transduce the effector function signal and direct the cell to perform a specialized function. The signaling domain can comprise signaling domains of other molecules. In some cases, a truncated portion of the signaling domain is used in a CAR. The intracellular signaling domain comprises multiple signaling domains involved in immune cell signaling, or any derivatives, variants or fragments thereof. The intracellular signaling domain of a CAR can comprise a costimulatory domain, e.g., from a costimulatory molecule.

In a specific embodiment, the CAR of the present application comprises or consists of the sequence of SEQ ID NO: 3, or a sequence with at least 75%, 80%, 85%, 90%, 95%, 97% or 99% homology thereto.

Binding of the CAR contained in the modified immune cell to the B cell surface protein can enhance immune cell proliferation, as compared to an immune cell lacking the CAR. Proliferation of the immune cell can refer to expansion or phenotypic changes of the immune cell. The modified immune cell of the invention comprising a CAR can bind to a B cell surface protein, and its proliferation can be about 5 times to about 10 times, about 10 times to about 50 times, about 50 times to about 100 times, about 100 times to about 200 times, about 200 times to about 300 times, about 300 times to about 400 times, about 400 times to about 500 times, about 500 times to about 600 times, or about 600 times to about 700 times that of the corresponding immune cell lacking the CAR. Proliferation can be determined at least about 12, 24, 36, 48, 60, 72, 84, or 96 hours after contacting the B cell with the modified immune cell comprising the CAR. Proliferation can be determined *in vitro* or *in vivo,* for example by determining the number of immune cells. The methods for determining the number of immune cells can comprise flow cytometry, Trypan Blue exclusion and/or hemocytometry. Proliferation can also be determined by phenotypic analysis of the immune cells.

The various domains of enhanced receptors and CARs provided herein can be linked by means of a chemical bond, e.g., an amide bond or a disulfide bond; a small organic molecule (e.g., a hydrocarbon chain); an amino acid sequence such as a peptide linker (e.g., an amino acid sequence of about 3-200 amino acids in length), or a combination thereof. The peptide linker can provide desirable flexibility, to enable the chimeric polypeptide to have the proper expression, activity and/or conformational positioning . The peptide linker can be of any suitable length to connect at least two domains of interest and is preferably designed to be sufficiently flexible so as to enable one or both of the domains it connects to properly fold and/or function and/or have activity. In some embodiments, the peptide linker has a length of about 0 to 200 amino acids, about 10 to 190 amino acids, about 20 to 180 amino acids, about 30 to 170 amino acids, about 40 to 160 amino acids, about 50 to 150 amino acids, about 60 to 140 amino acids, about 70 to 130 amino acids, about 80 to 120 amino acids, or about 90 to 110 amino acids. In some embodiments, the linker sequence can comprise an endogenous protein sequence. In some embodiments, the linker sequence comprises glycine, alanine and/or serine amino acid residues. In some embodiments, the linker can have GS, GGS, GGGGS, GGSG, or SGGG as the motif and contain multiple such motifs, e.g., multiple repeating motifs. The linker sequence can comprise any naturally occurring amino acids, non-naturally occurring amino acids, or combinations thereof.

In some embodiments, the modified immune cell of the invention also comprises a modification for regulating the immune cell death, such as a suicide switch. A suicide switch can be activated to eliminate the immune cell in cases of severe toxicity, such as hypercytokinemia. When the immune system has too strong response so that many inflammatory cytokines are released, triggering mild to severe symptoms including fever, headache, rash, rapid heartbeat, low blood pressure, and trouble breathing, the suicide switch can be initiated. The suicide switch can be a drug-inducible suicide switch. The suicide switch can comprise an inducible caspase 9.

In a preferred embodiment, the modified immune cell of the invention does not comprises an exogenously introduced TCR.

Any suitable delivery method can be used for introducing desired compositions and molecules (e.g., polypeptides, and/or nucleic acids such as nucleic acids encoding the polypeptides) into an immune cell to accomplish the modification. Various components may be delivered simultaneously or separately. The delivery method may comprise introducing into the immune cell one or more nucleic acids comprising a nucleotide sequence encoding the composition of the invention, for example the nucleic acids may be an expression vector, such as a recombinant expression vector, comprising a nucleotide sequence encoding a product of interest. Any suitable vector compatible with the host cell can be used in the invention.

Non-limiting examples of delivery or transformation methods include, but are not limited to, viral or bacteriophage infection, transfection, conjugation, protoplast fusion, lipofection, electroporation, calcium phosphate precipitation, polyethyleneimine (PEI)-mediated transfection, DEAE-dextran-mediated transfection, liposome-mediated transfection, particle gun technology, calcium phosphate precipitation, direct micro injection, and nanoparticle-mediated nucleic acid delivery.

Virus-based and non-virus-based gene transfer methods can be used to introduce nucleic acids into mammalian cells or tissues, and thus can be used in the invention to introduce nucleic acids of interest into cells in culture. Non-viral vector delivery systems can include DNA plasmids, RNAs (e.g., transcripts), naked nucleic acids, and nucleic acids complexed with delivery vehicles, such as liposomes. Viral vector delivery systems can include DNA and RNA viruses, which can be either integrated or not integrated with the genome of a cell after delivery to the cell.

RNA or DNA virus-based systems can be used to target specific cells in the body and trafficking the viral load to the nucleus of the cell. Viral vectors can be administered directly (*in vivo*) or used to treat cells *in vitro,* and can optionally be administered (*ex vivo*) to cells. Virus based systems can include retroviral, lentiviral, adenoviral, adeno-associated viral and herpes simplex viral vectors for gene transfer. Integration with the host genome can occur using the retrovirus, lentivirus, and adeno-associated virus gene transfer methods, which can result in long term expression of the inserted transgene. High transduction efficiencies can be observed in many different cell types and target tissues. Preferably, a lentiviral vector is used in the invention.

In some aspects, the invention provides methods for modifying an immune cell, comprising delivering one or more polynucleotides, or one or more vectors as described herein, or one or more transcripts thereof, and/or one or more proteins transcribed therefrom, to the immune cell. In some aspects, the disclosure also provides modified immune cells produced by these methods, and comprises these immune cells.

Target cells

The modified immune cells of the invention can target and act on a variety of target cells *in vitro, in vivo,* or *ex vivo.* The target cell may be an isolated cell. The target cell can be a cell inside of an organism. The target cell can be an organism. The target cell can be a cell in a cell culture.

The target cell can be or can be derived from a mammalian cell, and the mammal can be a rodent, or a primate such as a human. The target cell can be or can be derived from a prokaryotic cell, such as a bacterial cell, and an archaeal cell, or can be or can be derived from a eukaryotic cell.

The target cell can be a cell infected with a pathogen which may be a microorganism, including but not limited to a bacterium, fungus, or virus. The target cell can be a host cell, such as a host cell of the pathogen. In a specific embodiment, the target cell is, for example, a cell infected with hepatitis virus, preferably hepatitis B virus, or a cell infected with human papilloma virus.

The target cell can be from a specific organ or tissue. The target cell can be a unicellular organism.

The target cells can be stem cells or progenitor cells, including but not limited to adult stem cells, embryonic stem cells, induced pluripotent stem cells (iPSCs), and progenitor cell such as cardiac progenitor cells, neural progenitor cells, and the like. The target cell can be a pluripotent stem cell.

The target cell can be a genetically modified cell. The target cell can comprise a target nucleic acid.

The target cell can be a diseased cell, such as a cell from a diseased subject. A diseased cell can have altered metabolic, gene expression, and/or morphologic features. The diseased cell can be a cancer cell or a virus-infected cell.

In a preferred embodiment, the target cell is a tumor cell, particularly a cancer cell. The tumor include a solid tumor and a hematological tumor, preferably a solid tumor.

Therapeutic use

The modified immune cells of the invention may be used for the treatment of tumors, in other words, with tumor cells as target cells. In some embodiments, the target cells form a tumor. Treatment with the modified immune cells of the invention may result in stable growth, non-progression and/or non-metastasis of the tumor. For example, "stable growth" means that the volume of one or more tumors increases by no more than 1%, 5%, 10%, 15%, or 20% over a period of time after treatment. In some embodiments, the period of time is at least about 1, 2, 3, 4, 5, 6, 7, 8, 9, 10, 11, 12 or more weeks, preferably at least about 1, 2, 3, 4, 5, 6, 7, 8, 9, 10, 11, 12 or more months, and more preferably at least about 1, 2, 3, 4, 5, 6, 7, 8, 9, 10 or more years. In some embodiments, treatment with the modified immune cells of the invention can reduce the size of a tumor or the number of tumor cells by at least about 5%, 10%, 15%, 20%, 25%, 30%, 35%, 40%, 45%, 50%, 55%, 60%, 65%, 70%, 75%, 80%, 85%, 90%, 95% or more. In some embodiments, the tumor is completely eliminated, or reduced below a level of detection. In some embodiments, a subject remains tumor-free for at least about 1, 2, 3, 4, 5, 6, 7, 8, 9, 10, 11, 12 or more weeks, preferably at least about 1, 2, 3, 4, 5, 6, 7, 8, 9, 10, 11, 12 or more months, and more preferably at least about 1, 2, 3, 4, 5, 6, 7, 8, 9, 10 or more years after treatment.

The number of modified immune cells of the invention administered to a subject, when used to treat a tumor, is between $10^4$ and $10^{10}$, between $10^4$ and $10^9$, between $10^5$ and $10^8$, or between $10^6$ and $10^7$. When the PD-1$^+$T cells of the invention comprise an expansion factor modification such as CD 19 CAR, the total amount of reinfusion can be appropriately lower than that of the PD-1$^+$T cells of the invention that do not comprise the modification, because CD19 CAR contributes to the expansion of reinfused immune cells in a patient. This effect is described in detail in Example 5. For example, where the PD-1$^+$T cells of the invention comprise an expansion factor modification such as a CD 19 CAR, the total number of reinfused cells may be between $10^4$ and $10^9$, between $10^5$ and $10^8$, or between $10^6$ and $10^7$. The modified immune cells of the invention may be reinfused to the subject one or more times, e.g., once, twice, three times, four times. The number of immune cells per reinfusion may be the same or different.

The modified immune cells of the invention may be used for treatment of diseases or symptoms related to viral infections, in other words, with virus-infected cells as target cells. In some embodiments, the immune cells of the invention, while treating cancer in a patient, can eliminate viruses, such as residual viruses after anti-viral treatment, from the patient. Thus, the modified immune cells and methods of the invention are particularly suitable for patients known to have been infected with a virus. The viral infection may or may not be associated with cancer. In further embodiments, the immune cells of the invention are used specifically to target virus-infected cells, treat virus-associated diseases, and/or eliminate viruses.

In a preferred embodiment, the viral infection is a hepatitis B virus infection. For example, the viral infection is a chronic hepatitis B virus infection, e.g., the liver is infected with hepatitis B virus for a period of more than six months. Diseases associated with chronic hepatitis B virus infection include cirrhosis and hepatoma.

In another preferred embodiment, the viral infection is a human papilloma virus (HPV) infection. More than 100 types of human papilloma virus have been found so far. According to the correlation with cancer, HPV is generally divided into carcinogenic high-risk type (including but not limited to type 16, type 18, type 31, type 45, type 52, and type 84) and non-carcinogenic low-risk type (such as condyloma acuminatum-causing type 6, type 11, type 42, type 43, and type 44). HPV can cause proliferation of squamous epithelium of human skin mucous membrane, leading to diseases of skin, respiratory tract, oral cavity, digestive tract, eye, anus and genitourinary system. Diseases associated with HPV include, but are not limited to, warts such as verruca vulgaris, flat warts and condyloma acuminatum, and tumors or cancers such as squamous cell carcinoma, cervical cancer, vulvar cancer, vaginal cancer, penile cancer, anal cancer, and oropharyngeal cancer.

Death of target cells can be determined by any suitable method, including, but not limited to, counting cells before and after treatment, or measuring the level of a marker associated with live or dead cells. Degree of cell death can be determined by any suitable method. In some embodiments, degree of cell death is determined with respect to the starting condition. For example, an individual can have a known starting amount of target cells, such as a starting cell mass of known size or circulating target cells at a known concentration. In such cases, degree of cell death can be expressed as a ratio of surviving cells after treatment to the starting population of cells. A variety of cell death assays are available, and can utilize a variety of detection methods. Examples of detection methods include, but are not limited to, cell staining, microscopy, flow cytometry, cell sorting, and combinations thereof.

When a tumor is subjected to surgical resection following completion of a therapeutic period, the efficacy of treatment in reducing tumor size can be determined by measuring the percentage of a resected tissue that is necrotic (i.e., dead). In some embodiments, the treatment is effective if the necrosis percentage of the resected tissue is greater than about 20%, e.g., at least about 30%, 40%, 50%, 60%, 70%, 80%, 90% or 100%. In some embodiments, the necrosis percentage of the resected tissue is 100%, that is, no living tumor tissue is present or detectable.

Exposing a target cell to a modified immune cell of the invention can be conducted either *in vitro* or *in vivo*. Exposing a target cell to a modified immune cell generally refers to bringing the target cell in contact with the modified immune cell and/or bringing them in sufficient proximity such that an antigen (e.g., a membrane-bound or non-membrane-bound antigen) of the target cell can bind to a receptor expressed in the immune cell comprising an enhanced receptor and/or CAR. Exposing a modified immune cell to a target cell *in vitro* can be accomplished by co-culturing the target cell and the modified immune cell. The co-culturing can be conducted with adherent cells or cell suspension. Target cells and modified immune cells can be co-cultured in various suitable types of cell culture media, for example with supplements, growth factors, ions, etc. Exposing a target cell to a modified immune cell *in vivo* can be achieved, for example, by administering the modified immune cell to a subject, for example a human, and allowing the modified immune cell to localize to the target cell via the circulatory system. In some cases, a modified immune cell can be delivered to the area where a target cell is localized, for example, by direct injection. The exposing can be performed for any suitable length of time, for example at least 1 minute, at least 5 minutes, at least 10 minutes, at least 30 minutes, at least 1 hour, at least 2 hours, at least 3 hours, at least 4 hours, at least 5 hours, at least 6 hours, at least 7 hours, at least 8 hours, at least 12 hours, at least 16 hours, at least 20 hours, at least 24 hours, at least 2 days, at least 3 days, at least 4 days, at least 5 days, at least 6 days, at least 1 week, at least 2 weeks, at least 3 weeks, at least 1 month or longer.

**EXAMPLES**

For a more complete understanding and application of the present invention, the present invention will be described in detail hereinafter with reference to the examples and the accompanying drawings, and the examples are intended only to illustrate the present invention and are not intended to limit the scope of the present invention. The scope of the present invention is specifically defined by the appended claims.

**Example 1. Verification of the tumor recognition characteristic of PD-1$^+$T cells in a cancer patient**

In order to verify the tumor recognition characteristic of PD-1$^+$T cells from peripheral blood of a cancer patient, tumor tissues derived from the same patient were processed to construct a Patient-Derived tumor Xenograft (PDX) mouse model. The specific process was as follows.

**I. Tumor transplantation**

1. The fresh tumor samples obtained from a colon cancer patient during the operation were transferred to a 60 mm sterile culture dish, and an appropriate amount of PBS containing 1% penicillin-streptomycin was added to clean the tumor.
2. Tumor samples were cut in the size of 3 mm $\times$ 3 mm $\times$ 3 mm with a sterilized surgical blade, and transplanted into NPI (genetic background: NOD-Prkdcem1Idmo- il2rgem2Idmo) male mice aged 6-8 weeks subcutaneously.
3. NPI mice were injected intraperitoneally with an anesthetic before transplantation. After anesthesia, the mice were scissored with sterilized ophthalmic scissors to cut an opening of about 1 cm on the lateral upper skin of the right hind limb, a tumor mass was pushed with ophthalmic forceps to a lateral subcutaneous site of the right hind limb, and then the surgical incision was closed.

**II. Tumor observation**

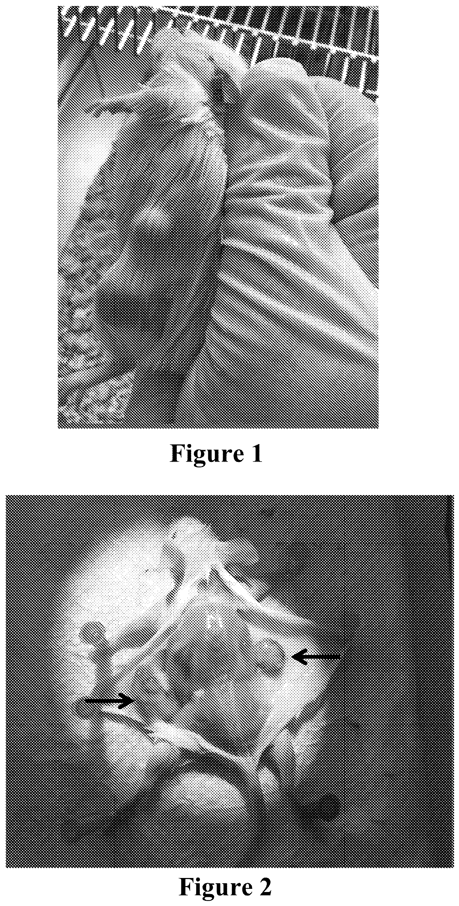

1. The tumor after tumor formation was P0 generation. The long diameter a and the short diameter b of the tumor were measured by a vernier caliper, and the tumor volume was calculated by formula (V) = 1 /2$\times$a$\times$b$^2$.
2. The tumor was removed when the P0 tumor grew to a size of 800-1000 mm$^3$, the necrotic tissues were removed, and the remaining portion was cut again in the size of 3 mm $\times$ 3 mm $\times$ 3 mm per piece, the above steps were repeated, and the tumor mass was transplanted to NPI mice subcutaneously for tissue culture passage. After tumor formation again, the tumor was marked as P1 generation (the tumor-bearing situation in the mouse was shown in Figure 1). These steps were repeated, and when culturing to the P3 generation, tumor tissues were removed from 3 mice respectively, for subsequent *in vitro* functional experiments (the anatomized tumor tissues were shown in Figure 2).
3. The tumor samples obtained in each generation can be used for genomic, proteomic and pathological analysis and the like. At the same time, slow-frozen samples can be retained and resuscitated for tumor-bearing use when there is a subsequent experimental need.

**III. Sorting of PD-1$^+$T and PD-1$^-$T cells**

When the PDX mouse model was cultured to the third generation, peripheral blood PD-1$^+$T cells and PD-1$^-$T cells from the same patient were sorted.

IIIa. PD-1$^+$T Cell Sorting

1. According to the estimated number and proportion of PD-1$^+$ cells to be sorted, the corresponding number of PBMC cells was taken and placed in a centrifuge tube;
2. Enough normal saline was added to resuspend and wash cells, and centrifuged at 600 g for 10 min, and the supernatant was discarded;
3. Step 2 was repeated, and the cells were washed with physiological saline a second time to obtain cell pellets;

4. 40 $\mu$l buffer per $1 \times 10^7$ total cells was added to resuspend the cells; 2 $\mu$l anti-PD-1-Biotin antibody (Biolegend, Cat#329934) was added per $1 \times 10^6$ effective cells, mixed well, and incubated at 4°C in dark for 10-15 min;

5. The mixture was washed with 0.5-1 ml buffer per $1 \times 10^7$ total cells, and centrifuged at 600 g for 10 min, and the supernatant was pipetted and discarded;

6. 80 $\mu$l buffer per $1 \times 10^7$ total cells was added to resuspend the cells, 20 ul anti-Biotin MicroBeads (Meltenyi, Cat#130-090-485) were added per $1 \times 10^7$ effective cells, mixed well, and incubated at 4°C in dark for 15 min;

7. The mixture was washed with 1-2 ml buffer per $1 \times 10^7$ total cells, and centrifuged at 600 g for 10 min, and the supernatant was pipetted and discarded;

8. 1 ml buffer was added per $1 \times 10^8$ total cells to resuspend the cells;

9. LS-type sorting column was placed on a magnetic frame, a 15 ml centrifuge tube was placed below, the column was rinsed once with 3 ml buffer, and then a new 50 ml centrifuge tube was used as a collection tube;

10. The resuspended cells in step 8 were mixed well, added into the LS-type column, and passed through the column, the column was rinsed with 3 ml buffer 3 times, and the unadsorbed cells, i.e. PD-1- PBMC cells, were collected, counted, and used for subsequent sorting of PD-1- T cells;

11. The LS-type sorting column was removed from the magnetic frame, and placed above a new 15 ml centrifuge tube;

12. 5 ml of buffer solution was added into the column, the liquid within the column was quickly pushed out with a piston, and the cells labeled by magnetic beads were collected as PD-1+ cells, and counted for later use;

13. $5 \times 10^5$ PD-1- and PD-1+ cells sorted in step 10 and step 12 were taken respectively and labeled with CD3-APC antibodies for flow cytometry detection;

14. According to the experimental needs, the corresponding number of PD-1+ cells was taken, and centrifuged at 600 g for 10 min, and the cells were resuspended with a complete medium;

15. Resuspended PD-1+T cells were activated by adding CD3/CD28 DynaBeads (Gibco, Cat#40203D) and subcultured.

IIIb. PD-1-T Cell Sorting

1. The proportion of T cells in PD-1-PBMCs was detected by CD3 flow cytometry, and the total number of T cells was calculated according to the cell count;

2. According to the number of T cells, and 1:3 of the ratio of T cells to CD3/CD28 DynaBeads, the corresponding number of CD3/CD28 DynaBeads were taken in a 15 ml centrifuge tube, and placed on the magnetic frame to remove the preservation solution;

3. The centrifuge tube was removed from the magnetic frame, a medium was added to resuspend the CD3/CD28 DynaBeads (10-fold volume), and the centrifuge tube was placed on the magnetic frame to remove the medium;

4. Step 3 was repeated twice, and CD3/CD28 DynaBeads were resuspended with 1-5 ml of medium for the last time;

5. 1/2 of the resuspended CD3/CD28 DynaBeads were added to a suspended cell solution, and the cell concentration was adjusted to $3.35-15 \times 10^6$/ml in a new centrifuge tube (15-50 ml for 50 ml centrifuge tube, and 2-15 ml for 15ml centrifuge tube);

6. The centrifuge tube was placed on a hybridization oven (1 rpm/min) and incubated at room temperature of 18-20°C for 30 min;

7. The centrifuge tube was carefully placed on the magnetic frame and stood for 2 min, and T cells bound to CD3/CD28 DynaBeads were collected, resuspended with an appropriate amount of medium, and counted;

8. The unadsorbed cell suspension was subjected to steps 5-7 again, and T cells bound to CD3/CD28 DynaBeads were collected a second time, resuspended with an appropriate amount of medium, and counted;

9. Unadsorbed cell suspension was counted and cryopreserved;

10. The T cells bound to CD3/CD28 DynaBeads that were collected in steps 7 and 8 were combined, the cell density was adjusted to $1.2 \times 10^6$/ml, and Tscm (T150 100-120 ml, T75 50-60 ml, T25 15-29 ml) was added; and

11. The mixture was mixed well and placed into a CO2 incubator for subculture.

**IV. Detection of T Cell Activation**

Tumor tissues were isolated from three P3-generation mice obtained from the above-mentioned Section II, cut into pieces, and prepared into single cell suspensions by enzymatic digestion method, which were named as tumor tissue 1#, tumor tissue 2#, and tumor tissue 3# respectively. The PD-1-T cells and PD-1+T cells prepared in the above-mentioned Section III were co-cultured with the 3 tumor tissue cells to form six cultures. At 24 h and 48 h, the six cultures were subjected to ELISPOT experiments to study the activation of different T cells by detecting IFN-$\gamma$.

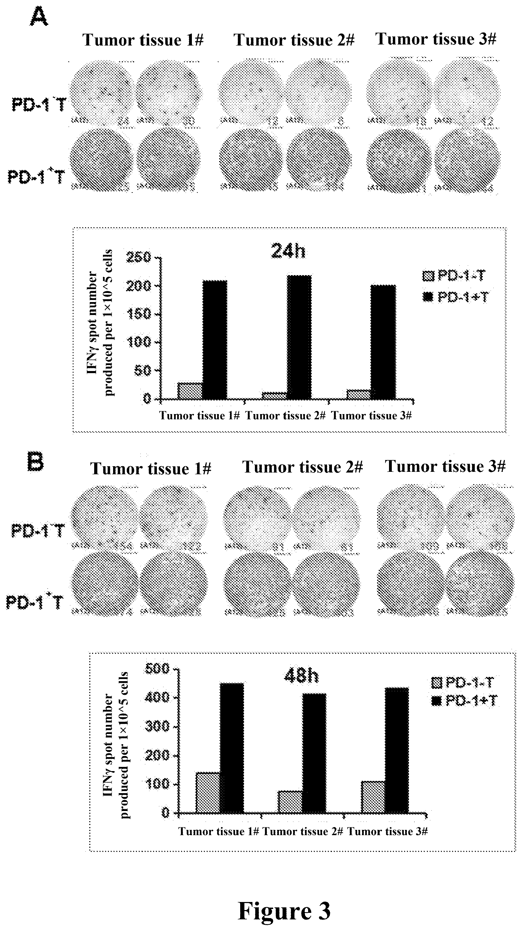

As shown in Figures 3A-B, IFN-$\gamma$ released by PD-1+T cells was significantly higher than that released by PD-1⁻T cells, implying that PD-1⁺T cells were activated by tumor tissue while PD-1⁻T cells were not. The results show that the PD-1⁺T cells obtained from the peripheral blood of the patient by the above sorting methods can effectively recognize tumor cells, generate immune response and initiate killing, indicating that there are T cells (tumor-specific T cells) in the peripheral blood of the patient that can exert immune function on tumor cells, and these cells are mainly in the PD-1⁺ component.

**Example 2. PD-1⁺T cell function improvement by knock-out of PD-1**

In order to verify whether the knock-out of PD-1 can effectively improve the function of PD-1⁺T cells by eliminating the "suppressive state" of PD-1⁺T cells, the experiment in this example was performed. The inventor constructed an *in vitro* model, in which tumor cells characterized by NY-ESO-1 expression were used and PD-1⁺TCR-T cells comprising the corresponding TCR capable of binding to NY-ESO-1 were constructed. On this basis, the CRISPR-Cas9 technology was used to knock out PD-1 gene in T cells to observe the changes in its effect on tumor cells.

First, the leukapheresis was performed for a patient, a PD-1 monoclonal antibody was added to sort the PD-1⁺T cells, and the sorting efficiency was shown in Figure 4A.

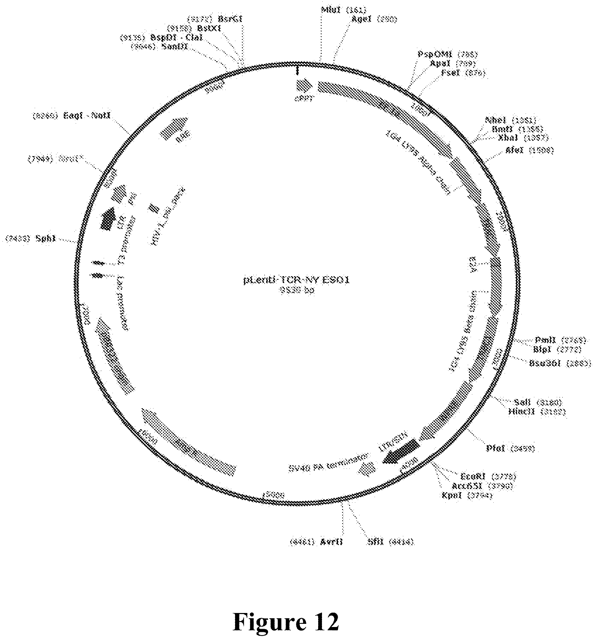

Subsequently, CD3/CD28 DynaBeads were added to the sorted PD-1⁺T cells and cultured overnight. On the second day, PD-1⁺T cells were divided into two groups: one group was NOTD-T without virus transfection, and the other group was TCR-T into which a lentivirus vector expressing TCR recognizing NY-ESO-1 (pLenti-TCR-NY ESO1, the plasmid structure as shown in Figure 12) was introduced, the lentiviruses being added at MOI (number of lentiviruses/number of cells) = 2. T cells were cultured and expanded, and the expression rate of the TCR was 40.1% as detected by flow cytometry (Figure 4B).

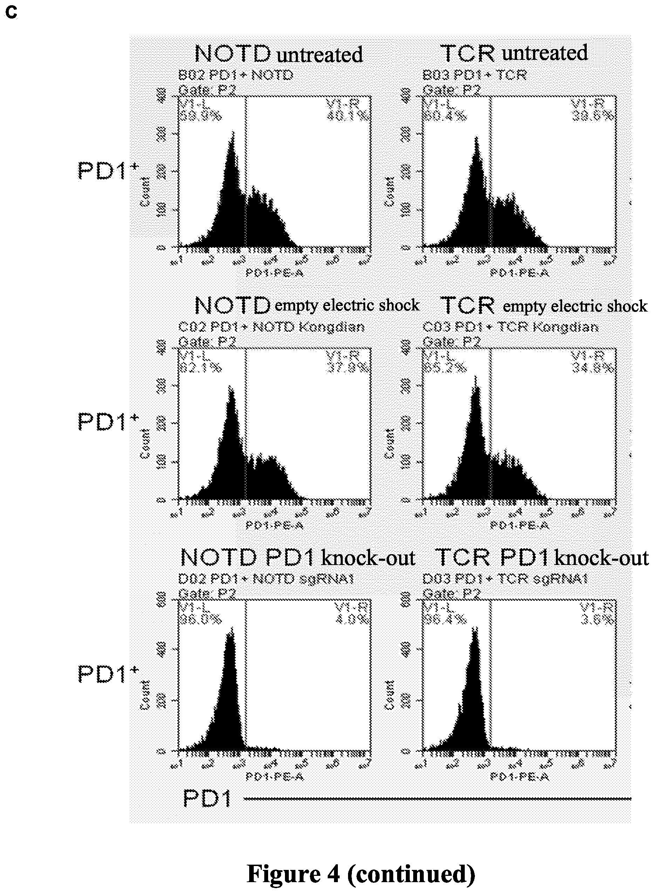

The CD3/CD28 DynaBeads were removed on the 4th day after the addition of lentivirus, and the PD-1 sgRNA and Cas9 protein were introduced into PD-1⁺NOTD-T cells and PD-1⁺TCR-T by means of electrotransfection on the 5th day to knock out the endogenously expressed PD-1, with untreated group and empty electric shock group as controls. As shown in Figure 4C, the expression of PD-1 was detected by flow cytometry on the 7th day after electrotransfection. The PD-1 positive proportions in the untreated group and empty electric shock group for NOTD-T cells were 39.6% and 34.8% respectively, and the PD-1 positive proportion decreased to 3.6% after knock-out of PD-1; the PD-1 positive proportion in the untreated group and empty electric shock group for TCR-T cells were 40.1% and 37.9% respectively, and the PD-1 positive proportion decreased to 4.0% after knock-out of PD-1. There was a good knock-out effect, and subsequent experiments can be performed.

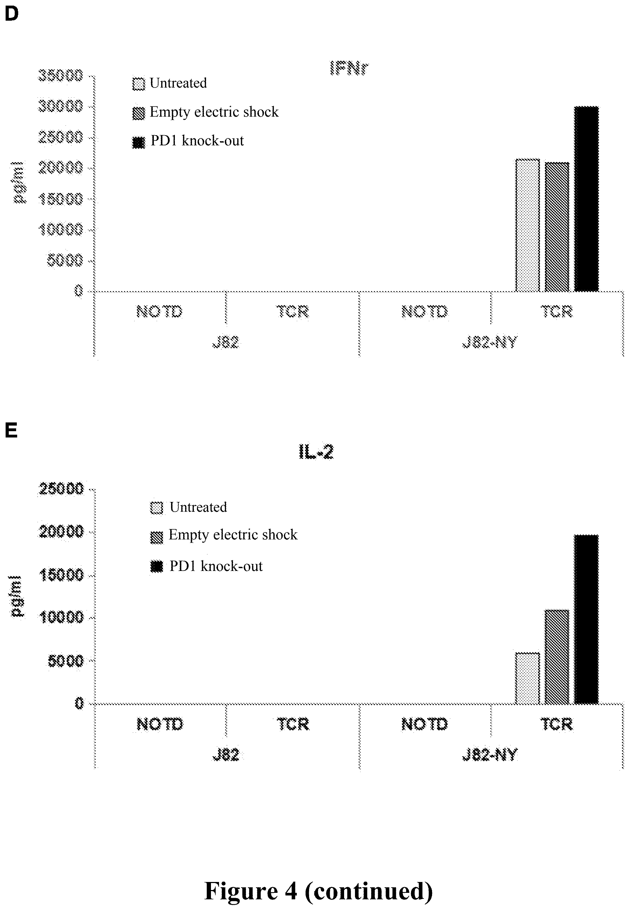

To verify the effect of knock-out of PD-1 on PD-1⁺T cell function, ELISA experiments were performed. Different groups of T cells were co-cultured with bladder cancer cell line J82 or J82-NY-ESO1 (J82-NY) tumor cells overexpressing NY-ESO-1 protein for 24 hours, and IFN-γ and IL-2 secretion was measured by ELISA. As shown in Figures 4D and 4E, knock-out of PD-1 can significantly promote the secretion of cytokines by PD-1⁺TCR-T cells. After being transfected with the virus vector expressing TCR and co-cultured with J82-NY-ESO1 tumor cells, IFN-γ secretion increased by nearly 50% and IL-2 secretion increased by not less than 80% for PD-1⁺T cells with knock-out of PD-1, as compared with two controls (untreated control and empty electric shock control), indicating that knock-out of PD-1 can effectively improve the immune efficacy of PD-1⁺T cells.

**Example 3. PD-1⁺T cell function improvement by an enhanced receptor *(in vitro* experiment)**

In cell therapy for solid tumors, in order to relieve the suppressive effect of inhibitory receptors such as PD-1 on immune cells and even further improve the potency of T cells, it was proposed that an enhanced receptor, comprising an extracellular domain capable of binding to a target cell of the immune cell and an intracellular domain of a T cell costimulatory molecule, can be introduced into T cells for the therapy, as described in the applicant's prior application PCT/CN2020/073017.

In order to verify whether an enhanced receptor has an effect on the immune efficacy of PD-1⁺T cells obtained by the method of the invention, the inventor constructed the following three PD-1⁺T cells comprising a TCR that targets the tumor target NY-ESO-1:

    1. PD-1⁺TCR-T cells, comprising a TCR that targets NY-ESO-1;

    2. PD-1⁺V1E-TCR-T cells, comprising an enhanced receptor V1E and a TCR that targets NY-ESO-1, wherein V1E comprises partial sequence of PD1 as the extracellular domain and comprises the transmembrane and intracellular domains of CD28, and the sequence of V1E is set forth in SEQ ID NO: 1;

    3. PD-1⁺V2E-TCR-T cells, comprising an enhanced receptor V2E and a TCR that targets NY-ESO-1, wherein V2E comprises an scFv antibody sequence capable of binding to PD-L1 protein as the extracellular domain and comprises the transmembrane and intracellular domains of CD28, and the sequence of V2E is set forth in SEQ ID NO: 2;

PD-1+NOTD-T cells without any TCR targeting NY-ESO-1 and any enhanced receptor were used as controls. The specific construction process was as follows:

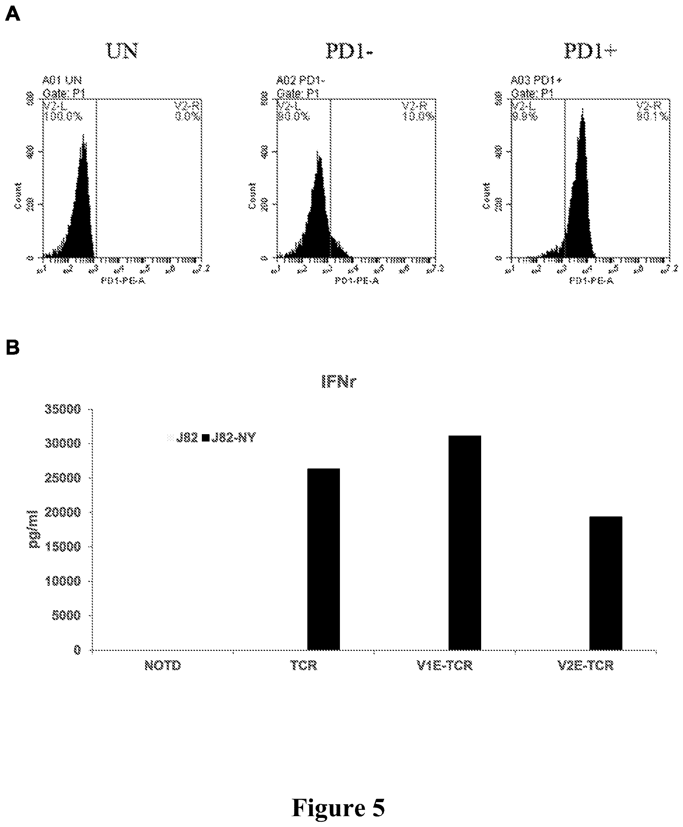

First, the leukapheresis was performed for a patient, a PD-1 monoclonal antibody was added to sort the PD-1+T cells, and the sorting efficiency was shown in Figure 5A. Subsequently, CD3/CD28 DynaBeads were added to the PD-1+T cells and cultured overnight. On the second day, PD-1+T cells were divided into 4 groups as follows:

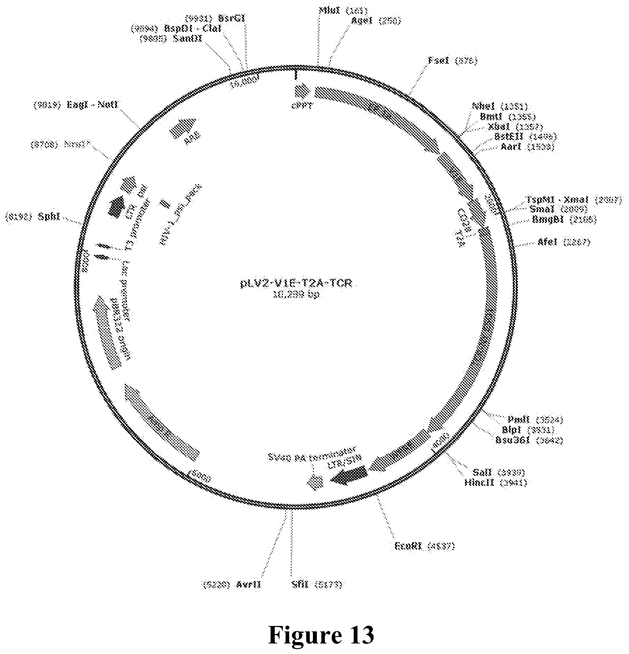

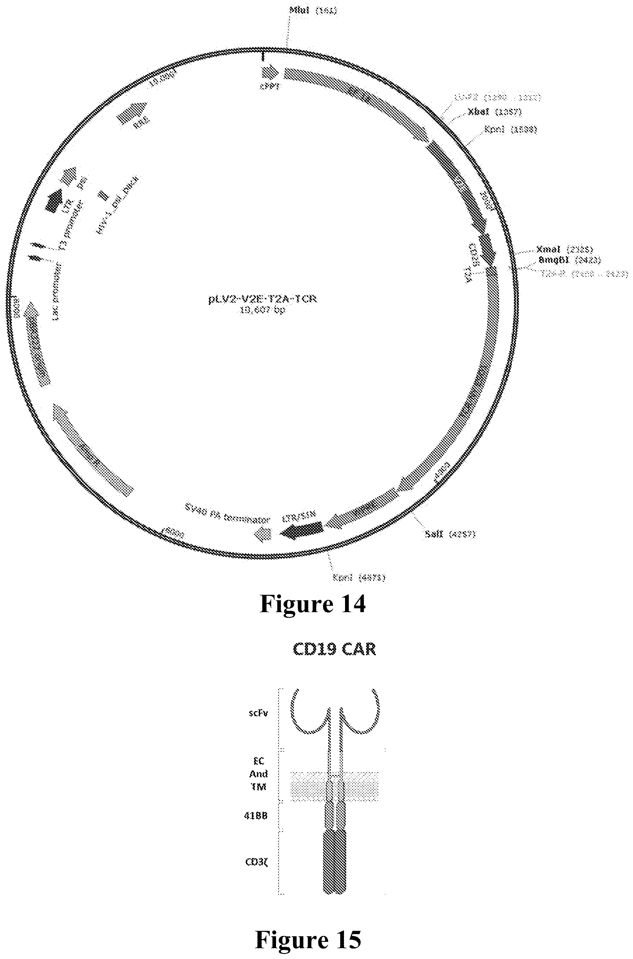

Group 1: NOTD-T, without virus transfection;
Group 2: TCR-T, to which a lentiviral vector expressing a TCR recognizing the NY-ESO-1 was introduced;
Group 3: V1E-TCR-T, to which a lentiviral vector expressing V1E-TCR was introduced, the vector structure being shown as in Figure 13; and
Group 4: V2E-TCR-T, to which a lentiviral vector expressing V2E-TCR was introduced, the vector structure being shown as in Figure 14.

For groups 2-4, lentiviruses were added at MOI (number of lentiviruses/number of cells) = 2, and T cells were cultured and expanded.

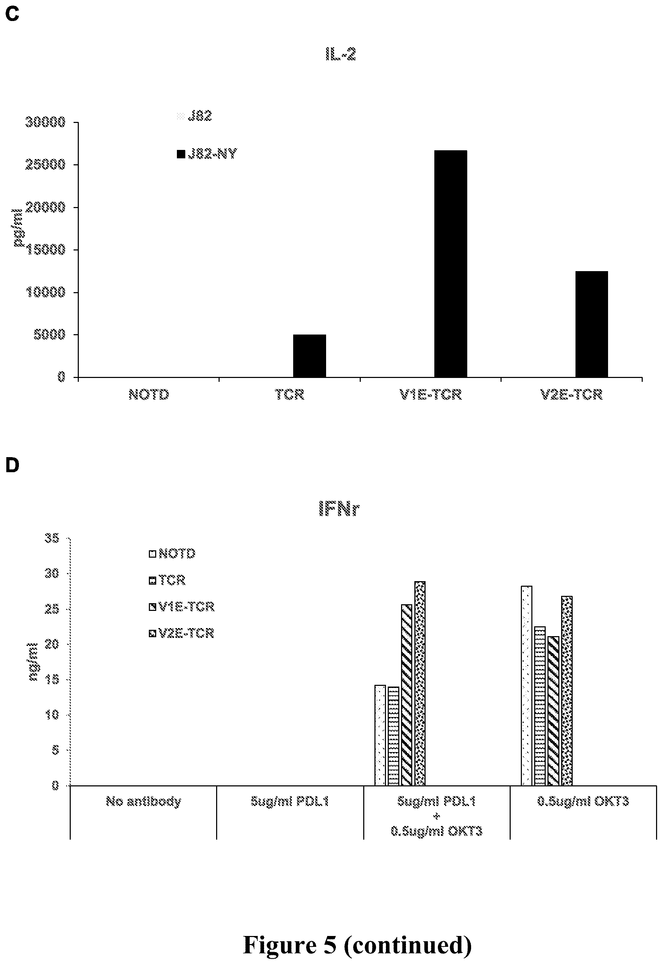

To verify the effect of an enhanced receptor on PD-1+T cell function, ELISA experiments were performed. Different groups of T cells were co-cultured with J82 tumor cell or J82-NY-ESO1 tumor cells overexpressing NY-ESO1 for 24 hours by the inventor, and IFN-$\gamma$ and IL-2 secretion was measured by ELISA. As shown in Figures 5B and 5C, the enhanced receptor V1E can significantly promote the IFN-$\gamma$ secretion by PD-1+TCR-T cells; and both the enhanced receptors V1E and V2E can significantly promote the IL-2 secretion by PD-1+TCR-T cells, indicating that both of the enhanced receptors can effectively improve the function of PD-1+T cells.

In order to further verify the effect of an enhanced receptor on the function of PD-1+T cells, the above-mentioned 4 groups of T cells were added to 96-well plates coated with OKT3 (a CD3 antibody), PD-L1 protein or OKT3+PD-L1 protein respectively, and cultured for 48 hours, and the supernatants were taken for ELISA detection of the IFN-$\gamma$ secretion. As shown in Figure 5D, OKT3 was able to effectively activate T cells to secrete IFN-$\gamma$. When costimulated with OKT3 and PD-L1 protein, the cells of the TCR-T and NOTD groups secreted a decreased level of IFN-$\gamma$, due to the inhibition of T cell function by PD-L1 protein binding to PD-1 protein on the surface of T cells, and this also simulated the inhibition of T cell function by PD-L1 on target cells. In contrast, not only were the function of T cells expressing the enhanced receptor not inhibited by the PD-L1 protein, but T cells expressing the enhanced receptor treated with both OKT3 and PD-L1 had significantly higher IFN-$\gamma$ secretion than treatment with OKT3 alone, indicating that the binding of the PD-L1 protein to the V1E or V2E enhanced receptor not only relieves the inhibition of T cells by the PD-L1 itself, but also significantly promotes cytokine secretion by T cells. This result indicates that expression of the enhanced receptor by the PD-1+T cell obtained by the invention can effectively convert the PD-L1 inhibitory signal into an activation signal for the T cell, thereby enhancing the function of the PD-1+T cell.

## Example 4. PD-1+T cell function improvement by an enhanced receptor (in vivo experiment)

In order to further verify the effect of an enhanced receptor on the tumor-inhibiting function of PD-1+T cells in vivo, two systems were constructed. One includes mice transplanted with a tumor cell line, and T cells expressing a TCR capable of recognizing the tumor cell line and an enhanced receptor; and the other includes tumor cells from a patient with a solid tumor and T cells from the same patient.

### 1. Tests using mice inoculated with J82-NY-ESO1 tumor cell line

Firstly, twenty NSG mice were inoculated with J82-NY-ESO1 tumor cells. When the average tumor volume reached 100 mm$^3$, 20 mice were randomly divided into 5 groups, 4 mice in each group.

At the same time, PD-1+T cells were sorted from the peripheral blood of a tumor patient, and the sorted negative components were defined as PD-1-T cells. Lentivirus transfection was performed on sorted PD-1+T and PD-1-T cells, wherein TCR (TCR recognizing NY-ESO1)-expressing and V1E-TCR-expressing lentiviruses were introduced at doses of MOI = 2, respectively, and PD-1-TCR-T, PD-1+TCR-T and PD-1+V1E-TCR-T cells were prepared as described in Example 3, with PD-1+NOTD cells (PD-1+T cells not transfected with viruses) as control.

After the completion of cell preparation, the cells were reinfused to 4 groups of mice, while PBS was reinfused to 1 group of mice as a blank control. The tumor volume in mice was calculated by measuring the length, width and height of a subcutaneous tumor at different time points after reinfusion.

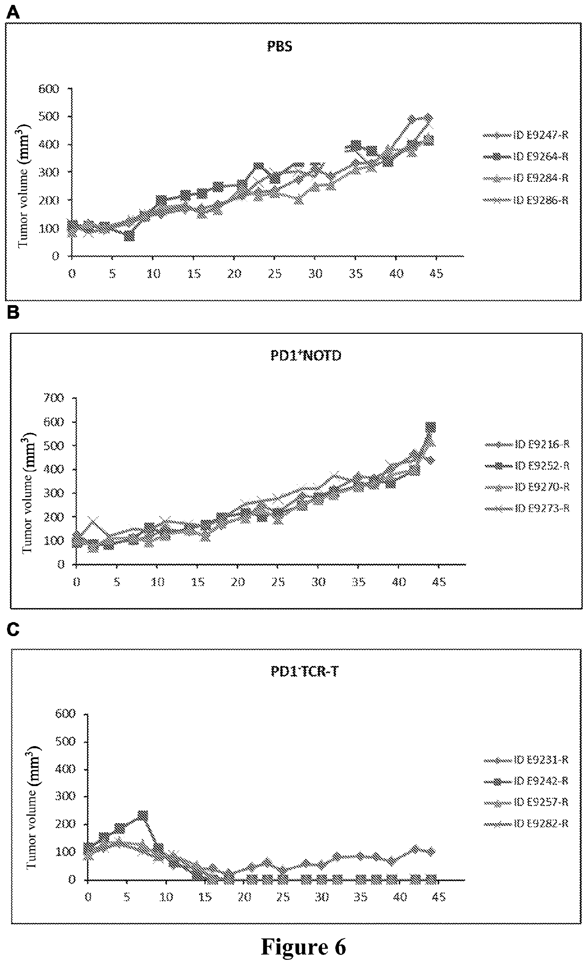

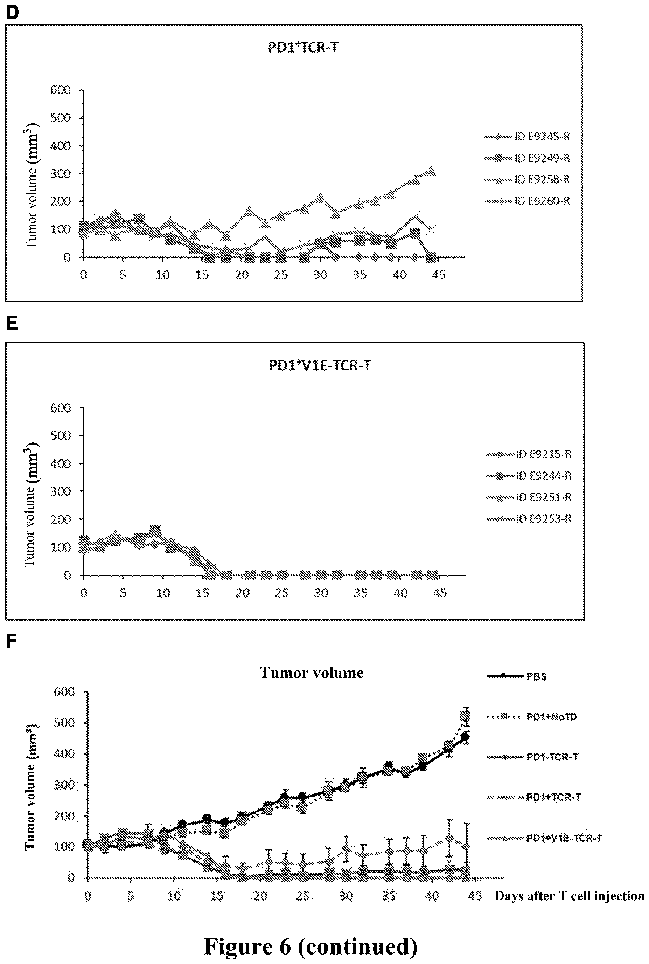

As a result, as shown in Figure 6, the tumors of mice reinfused with PBS and PD-1+NOTD cells continued to proliferate (Figures 6A and 6B), and the tumor volume reached about 500 mm$^3$ on day 44 after T cell reinfusion. The other three groups of mice reinfused with PD-1-TCR-T, PD-1+TCR-T and PD-1+V1E-TCR-T cells had reduced tumor volumes (Figures 6C-E), and the effect of PD-1+V1E-TCR-T cells was the most obvious, in which the tumor was basically completely removed.

The mean tumor volume curve for each group was shown in Figure 6F. The mean tumor volumes of mice of groups reinfused with PD-1$^-$TCR-T, PD-1$^+$TCR-T and PD-1$^+$V1E-TCR-T cells were 25.43 mm$^3$, 102.4 mm$^3$ and 0 mm$^3$, respectively, all showing a good tumor-inhibiting effect.

As mentioned above, PD-1$^+$V1E-TCR-T cells had the strongest tumor-inhibiting effect, wherein tumors of 4 mice were completely eliminated, achieving 100% CR efficacy. This efficacy was better than the tumor-inhibiting effect of PD-1$^-$TCR-T, indicating that the enhanced receptor loaded in PD-1$^+$T can effectively improve the function of PD-1$^+$T cells, so that the depletion state of PD-1$^+$T cells, which was not as effective as PD-1$^-$T cells in the tumor-inhibiting effect, was reversed, resulting in a better effect than PD-1$^-$T cells that did not express the enhanced receptor.

2. Tests using mice transplanted with patient tumor tissues

Similar experiments were conducted using mice transplanted with tumor tissues from a patient and T cells from the same patient.

The inventor constructed a PDX (Patient-Derived Tumor Xenograft) model using tumor tissues from a patient with a solid tumor. When the PDX model was subcultured to the 5th generation, 20 mice were selected. When the average tumor volume reached 100 mm$^3$, 20 mice were randomly divided into 4 groups, 5 mice in each group.

At the same time, PD-1$^+$T cells were sorted from the peripheral blood of the same patient, and the sorted negative components were defined as PD-1$^-$T cells. A part of the sorted PD-1$^+$T cells were transfected with lentiviruses, wherein the lentiviruses expressing the enhanced receptor V2E were introduced at a dose of MOI = 2 (the construct was the same as the lentivirus vector expressing V2E-TCR in Example 3, except that it did not comprise the fragment encoding TCR), and PD-1$^-$T, PD-1$^+$-T and PD-1$^+$V2E-T cells were prepared.

After the completion of cell preparation, the cells were reinfused to 3 groups of mice, while PBS was reinfused to 1 group of mice as a blank control. The tumor volumes in mice were determined at different time points after reinfusion.

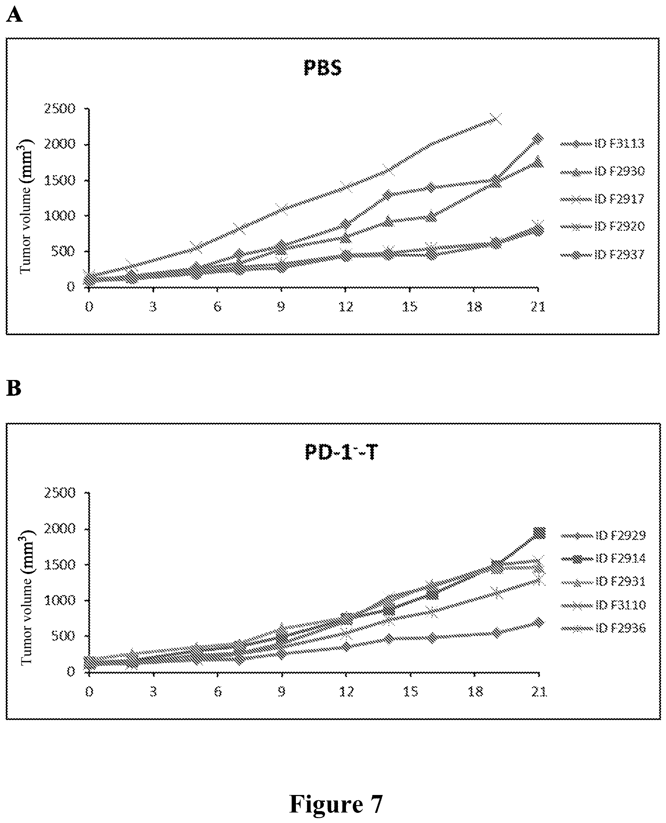

The tumor proliferation of different mice in each group was compared, and the results were shown in Figures 7A-C. Since the tumor tissues from the patient was directly transplanted, compared with the cell line used above, the tumor tissue transplanted into each mouse may have greater heterogeneity in cell composition, so that there were some differences in the results between different mice in the same group. Therefore, in this experiment, the average value of each group was not calculated and compared as in the previous experiment.

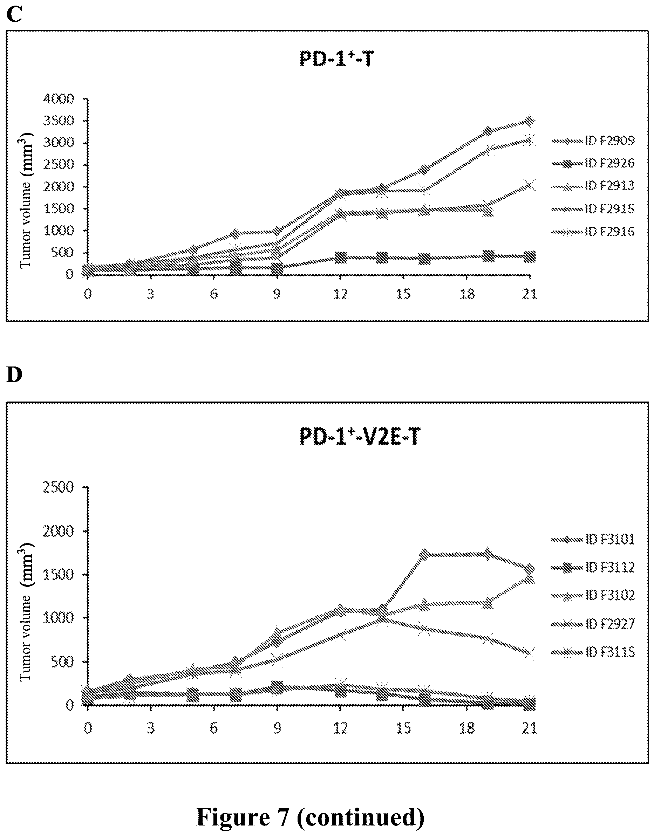

In spite of this, it can also be seen from Figure 7 that all the tumors of mice reinfused with PBS, PD-1$^-$T and PD-1$^+$-T cells continued to proliferate, and the proliferation rates were relatively fast, and no tumor-inhibiting effect was seen. In contrast, in mice reinfused with PD-1$^+$V2E-T cells, 3 out of 5 mice showed a shrinkage in tumor volume, in which the tumor volumes of two mice decreased from the initial 94 mm$^3$ and 85 mm$^3$ to 21 mm$^3$ and 52 mm$^3$, respectively (Figure 7D).

The results show that loading the enhanced receptor in PD-1$^+$T cells can effectively improve the function of PD-1$^+$T cells, even reverse the state of PD-1$^+$T cells, and promote their tumor-inhibiting ability *in vivo*.

**Example 5. First batch of clinical trials of PD-1$^+$T cells modified by an enhanced receptor and CAR**

In the context of the present application, PD-1$^+$T cells in the peripheral blood of patients with advanced tumors are considered to be T cells that have ever infiltrated into the tumors and returned to the peripheral blood after being suppressed by the tumor microenvironment, and are referred to as cTILs (circulating tumor infiltrating lymphocytes).

9 patients (or subjects) with advanced solid tumors who failed to respond to traditional treatment were recruited in a clinical trial research. PBMCs of patients were collected, and PD-1$^+$T cells (cTILs) therein were isolated, and subjected to gene modification, which allowed the cells to express the enhanced receptor V1E and expansion factor (CD19 CAR, the sequence as set forth in SEQ ID NO: 3), to prepare super cTILs (ScTILs) which were then reinfused to the patients. The specific trial was as follows.

**1. Screening of subjects**

The clinical situations of the patients before treatment were shown in the table below.

**Table 1.** Clinical situations of patients

| Number | Gender | Age | Tumor Type | Clinical Stage | TMB |
|--------|--------|-----|------------|----------------|-----|
| 1 | Male | 54 | Gallbladder carcinoma | Phase IV | Medium |
| 2 | Female | 54 | Ovarian carcinoma | Phase IV | Medium |

(continued)

| Number | Gender | Age | Tumor Type | Clinical Stage | TMB |
|---|---|---|---|---|---|
| 3 | Female | 50 | Lung cancer | Phase IV | Low |
| 4 | Male | 73 | Colon cancer | Phase IV | High |
| 5 | Female | 53 | Pancreatic neuroendocrine tumor | Phase IV | Medium |
| 6 | Male | 63 | Lung cancer | Phase IV | Low |
| 7 | Male | 52 | Kidney cancer | Phase IV | Medium |
| 8 | Female | 61 | Mucosal melanoma | Phase IV | Low |
| 9 | Male | 57 | Pancreatic cancer | Phase IV | Medium |

In the above table, the TMB represents tumor mutation burden, evaluated as the number of mutations per million bases in the genome, and a TMB below 5 indicates a low mutation burden, a TMB of 5-10 indicates a medium mutation burden, and a TMB above 10 indicates a high mutation burden. It is generally recognized in the art that a higher TMB is more suitable for immunotherapy and a lower TMB is less suitable for such therapy.

As can be seen from the above table, the recruited patients were solid tumor patients with eight different types of cancer (gallbladder carcinoma, ovarian carcinoma, lung cancer, colon cancer, pancreatic neuroendocrine tumor, kidney cancer, mucosal melanoma, and pancreatic cancer), all of them were at advanced stage and failed to respond to traditional multiple line treatment, with multiple distant metastatic foci in the body, and most patients had not high TMB levels.

## 2. Cell preparation before treatment

2.1 Isolation of PD-1$^+$T (cTIL) cells:

(1) Performing apheresis to isolate autologous peripheral blood mononuclear cells (PBMCs) from the patients. A flow cytometer was used to detect the proportion of PD-1$^+$T cells (PD-1$^+$CD3$^+$double positive cells) in total T cells (CD3$^+$ cells) (see the table below). The proportion of PD-1$^+$T cells in total T cells was identified as 28.5% (patient 1) and 14.3% (patient 2) (Table 2), and such a high proportion of PD-1$^+$T cells was considered as TILs from tumor tissues, which passed from tumor tissue into peripheral blood and were thus named as cTILs (circulating TILs).
(2) Sorting PD-1$^+$T cells by the method described above.

2.2 Preparation of ScTILs:

Super cTILs (ScTILs) were prepared by transfecting cTIL cells with a combination lentivirus simultaneously loaded with V1E and CD19 CAR. The lentiviral transfection process was as described in Example 3. After 8-10 days of natural expansion, the proportion of CAR$^+$ cells (considered as effective cells) in the cells was detected by flow cytometry, which showed that the proportion of effective cells was 10%-40%. After the quality control release inspection, the cells were put into an infusion bag. The entire preparation process was only 8-10 days (without the process of identification of T cells recognizing neoantigen components).

## 3. Reinfusion treatment and monitoring:

All patients received intravenous reinfusion of ScTILs. Translucent, light yellow ScTIL cell suspension for intravenous reinfusion was resuscitated at the bedside in a 38.5°C water bath and then rapidly infused intravenously. The reinfusion volume was determined according to the total cell number. The total cell volume was $6.9 \times 10^5$-$4.5 \times 10^8$, thereinfusion volume was 20-40 ml, the cell density was about $3 \times 10^6$-$3 \times 10^7$/ml, and the total effective cell (CAR positive cell) reinfusion number was shown in Table 2. Patients after reinfusion were hospitalized for continuous observation.

3.1 Assessment of safety

Of all the 9 subjects, only 3 had high fever, one of whom relieved spontaneously, and the other 2 had the symptom relieved after treatment with tocilizumab. The incidence rate of cytokine storm (cytokine release syndrome, CRS) was 33% (3/9), all being grade 1 CRS, and the risk of CRS was far lower than that of CD19 CAR-T treatment of hematological tumors. None of the patients developed autoimmune diseases after treatment.

It can be seen that the treatment regimen of the invention in which cTIL cells are modified with the expansion factor CD19 CAR and the enhanced receptor and reinfused to the subject does not produce uncontrollable severe side effects, with ideal safety.

3.2 Assessment of efficacy

The results of the imaging examination of the baseline before reinfusion and the imaging examination two months after cell reinfusion showed that the disease of all subjects were effectively controlled in the observation period of six weeks after cell reinfusion, indicating that the disease control rate of ScTILs of the present application for advanced solid tumors was about 100% (9/9), that is, the response rate of patients to treatment was 100%, in which three of the patients had significant tumor shrinkage or loss of tumor activity, i.e., an objective response rate was 33% (3/9).

**Table 2.** Treatment outcomes of patients

| Number | Gender | Age | Tumor Type | Clinical Stage | TMB | Proportion of PD-1$^+$T cells in total T cells | Dose of effective cells (CAR$^+$ cells) | Efficacy | |
|---|---|---|---|---|---|---|---|---|---|
| 1 | Male | 54 | Gallbladder carcinoma | Phase IV | Medium | 28.5% | $5.1\times10^5$ | SD | Tumor no longer progresses |
| 2 | Female | 54 | Ovarian carcinoma | Phase IV | Medium | 14.3% | $9.8\times10^4$ | PR | Tumor shrinkage |
| 3 | Female | 50 | Lung cancer | Phase IV | Low | 9% | $1.5\times10^7$ | SD | Tumor no longer progresses |
| 4 | Male | 73 | Colon cancer | Phase IV | High | 28% | $1.0\times10^8$ | SD | Tumor no longer progresses |
| 5 | Female | 53 | Pancreatic neuroendocrine tumor | Phase IV | Medium | 30% | $6.6\times10^6$ | SD | Reversal from end-stage |
| 6 | Male | 63 | Lung cancer | Phase IV | Low | 12% | $2.1\times10^6$ | SD | Tumor no longer progresses |
| 7 | Male | 52 | Kidney cancer | Phase IV | Medium | 18% | $1.0\times10^7$ | SD | Tumor no longer progresses |
| 8 | Female | 61 | Mucosal melanoma | Phase IV | Low | 23% | $3.1\times10^6$ | PR | Tumor shrinkage |
| 9 | Male | 57 | Pancreatic cancer | Phase IV | Medium | 40% | $1.8\times10^8$ | CR | Loss of tumor activity |

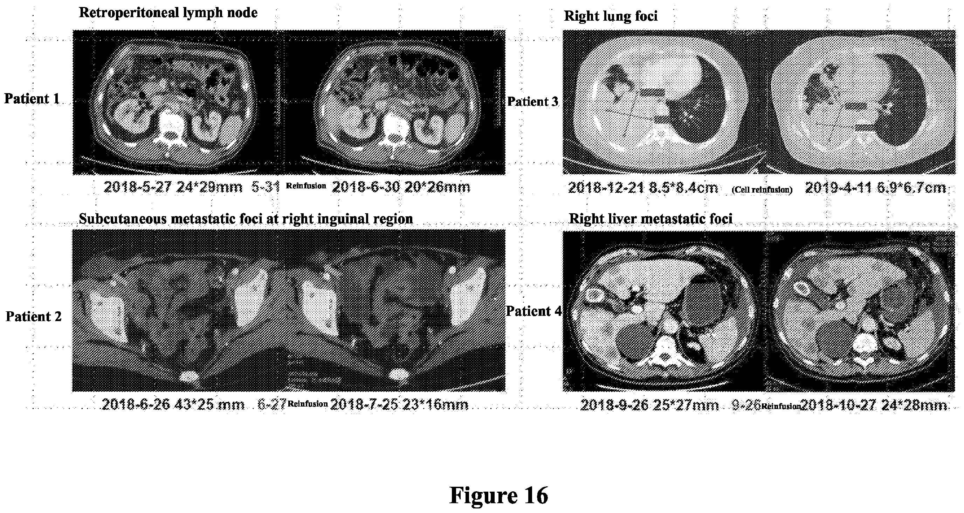

The efficacy evaluation in the rightmost column of Table 2 was performed using RECIST criteria. The imaging data of patients 1-4 are shown in Figure 16, and the foci are shown as arrows or scales. As shown in the figure, the tumor foci were significantly shrunken after treatment.

It can be seen that the PD-1+T cells modified by the expansion factor CD19 CAR and the enhanced receptor of the invention can effectively control or even treat a variety of different solid tumors, and have a wide range of potential application lineages; the effect is not related to the patient's tumor mutation burden, and thus the application is not limited to patients with high TMB.

3.3 CAR facilitates verification of ScTILs' *in vivo* expansion characteristics

At different time points (30 minutes, 24 hours, 4 days, 7 days, 10 days, 14 days, 28 days, 71 days and 91 days) after reinfusion, peripheral blood was taken for flow cytometry to detect the ratio of the number of CAR positive cells to the number of lymphocytes, thus dynamically monitoring the proportion of CAR positive cells (i.e. ScTIL cells) in the peripheral blood of patients 1 and 2, and calculating the absolute value of the number of these cells in patients. The expansion fold of ScTIL cells in the peripheral blood was calculated by the following formula:

$$\text{Expansion fold} = [\text{lymphocyte concentration (number/L)} \times \text{circulating blood volume (L)} \times \text{proportion of ScTILs in lymphocytes}]/\text{number of reinfused ScTIL cells}$$

The data showed that the CAR copy number in the peripheral blood 30 minutes after cell reinfusion was significantly higher than the number of reinfused effective cells, suggesting that the cells began to expand rapidly immediately after reinfusion. Therefore, the number of reinfused cells can be directly used as the baseline number in the above calculation formula, and the peripheral blood CAR value detected through blood collection 30 minutes after reinfusion can no longer be used as the baseline value without expansion.

Among them, the lymphocyte concentration can be detected by blood routine test; and the proportion of ScTILs in T cells can be obtained by flow cytometry to detect the proportion of CAR+ cells in CD3+ cells. The total circulating blood volume was converted based on the weight value in kilogram before cell reinfusion treatment. The total blood volume (liter/L) was calculated according to 80 ml/kg body weight for men and 75 ml/kg body weight for women.

After calculation, on the 14th day (D14) after reinfusion of ScTILs, patients 1 and 2 were randomly selected to detect and calculate the expansion fold of ScTIL cells in the peripheral blood, respectively, which was found to be 140-fold and 754-fold respectively. At the same time, it was found that the number of peripheral blood B cells was greatly reduced to 8%-25% of that before reinfusion (reduced by 75%-92%).

**Table 3.** Changes in the number of ScTIL cells and B cells 14 days after reinfusion

| Patient number | The number of reinfused ScTIL cells | The number of ScTIL cells in the peripheral blood at D14 | Expansion fold | The number of B cells before reinfusion | The number of B cells at D14 | B cell reduction percentage |
|---|---|---|---|---|---|---|
| 1 | $5.1 \times 10^5$ | $7.07 \times 10^7$ | 140 | $3.53 \times 10^8$ | $8.77 \times 10^7$ | 75% |
| 2 | $9.8 \times 10^4$ | $7.39 \times 10^7$ | 754 | $3.02 \times 10^8$ | $2.39 \times 10^7$ | 92% |

None of the patients developed immunodeficiency, even though no exogenous immunoglobulin was administered during the observation period.

It can be seen that cTIL cells with modification of expansion factor CD19 CAR in the invention can effectively facilitate themselves to expand *in vivo* by means of CD19 CAR recognition of B cells.

3.4 Verification of dual recognition ability of ScTILs

The scTILs in the invention was collected from peripheral blood-derived TILs (cTILs), and these T cells had the property of naturally recognizing tumors, i.e., the first recognition ability of T cells. In addition, on the basis of ScTILs, introducing the design of CD19 CAR targeting B cells can realize the great expansion of ScTILs *in vivo* by means of the stimulation of B cells to its target CD19.

Patients 1 and 2 (Table 1 and Table 2) were randomly selected, and the number of circulating tumor cells (CTCs) in the peripheral blood was dynamically monitored to compare the baseline value on the day of reinfusion and the value reexamined within two months after reinfusion. The results showed that the number of CTCs per 5 ml of peripheral blood decreased significantly in both subjects two months after reinfusion of ScTIL cells, as shown in the table below.

**Table 4.** Changes in the number of CTCs after treatment

| Subject number | Baseline CTC number/5 ml before treatment | CTC number/5 ml 60 days after treatment | Reduction percentage |
|---|---|---|---|
| 1 | 37 | 1 | 97% |
| 2 | 9 | 3 | 67% |

It can be seen that the cTIL cells with the expansion factor CD19 CAR of the invention can not only recognize B cells by CD19 CAR to cause large-scale *in vivo* amplification of TILs or tumor-recognizing T cells, but also further recognize and kill tumor cells in tumor patients by virtue of the natural recognition property of the cells themselves for tumor cells. This dual recognition ability of the modified immune cells of the invention was successfully confirmed by the clinical trial described above.

### 4. Efficacy evidence and analysis

The disease control rate of ScTIL therapy in this example was 100%; however, if only the natural TIL cells were reinfused, it was not enough to produce such efficacy, for the following reasons:

1) The number of reinfused cells was not sufficient to produce such efficacy. Even based on the number of T cells detected in the peripheral blood after expansion ($10^5$-$10^6$/kg body weight), in conjunction with relevant literature reports of previous cell therapies (such as Steven Rosenberg's report on neoantigen reactive T cells or TIL therapy, the number of reinfused cells was about $10^{10}$ to $10^{11}$), this dose was lower than the conventional reinfused dose; therefore, such dose was not sufficient to produce the efficacy observed in this example in the treatment of solid tumors.

2) The ScTILs was derived from TILs, which were usually depleted T cells. Without genetic modification, the effect of TILs on killing tumors was very limited, and reinfusion of depleted T cells at such a low dose was not sufficient to produce the efficacy of this example.

Therefore, according to the efficacy data of this example, it can be demonstrated from another perspective that the application of the enhancer receptor to the PD-1$^+$T cells in the invention can enhance the ability of these peripheral blood-derived immune cells to suppress tumors.

### 5. Summary

Combining the results of a number of experiments in this example, it can be concluded that the ScTIL therapy of the invention has the following characteristics:

1) PD-1$^+$T cells (cTILs) can be obtained from easily available peripheral blood by simple sorting, so that tumor-recognizing T cells can be obtained quickly and the preparation cycle is shortened;

2) The design of the enhanced receptors allows super cTIL (ScTIL) cells to change from a suppressive state to an activated state, thereby enhancing tumor killing efficiency.

3) The design of CARs targeting B cells (CD19) allows for large expansion of ScTIL cells *in vivo,* so that only a few ScTIL cells need to be prepared and large expansion *in vitro* is not required.

4) The cell preparation process is simple and fast, without the cumbersome steps of gene sequencing, screening, synthesis and the like in the traditional neoantigen treatment technology, thereby greatly shortening the preparation cycle and further greatly reducing the preparation cost.

5) The ScTIL therapy of the invention shows very good efficacy for solid tumor patients with different cancer types who failed to respond to traditional multiple line treatment, and has potential wide application.

6) The ScTIL therapy of the invention has ideal safety: a) there are no severe off-target or autoimmune diseases and other toxic side effects; b) only slight cytokine storm (cytokine release syndrome, CRS) occurs, which is far lower than CRS grade in CAR-T treatment of B cell hematological tumors; c) although there is some killing of normal B cells, its side effects will not be stronger than the application of CAR-T to treat B cell hematological tumors. This is because after CD19 CAR-T treatment of B-lineage hematological malignancies, B-cell deficiency usually lasts for no less than 18 months, and some patients even need the immunoglobulin replacement therapy for life. In contrast, B cells will recover and stabilize at levels close to or reaching the lower limit of the normal physiological range within 1-3 months after ScTIL treatment, and during this period, no immunoglobulin was found to be below the lower limit of normal physiological levels in all subjects.

**Example 6. Enhanced receptor-modified PD-1⁺T cells to treat malignant solid tumors with incidental elimination of residual viruses of previous HBV infection**

In clinical trials using ScTIL cells to treat patients with solid tumors, the inventor was surprised to observe that the patients' hepatitis B markers and liver enzymes were abnormal after treatment with the modified immune cells of the invention (ScTILs). Based on this observation, the inventor has speculated and designed experiments for verification, and then proposed that the immune cells of the invention can be used to treat diseases related to HBV infection, as well as to treat residual HBV viruses in the body.

1. Hepatitis B marker and liver enzyme abnormalities occurred after ScTIL cell therapy: properties and causative factors

In clinical trials, the following phenomena were observed in two patients outside the group after treatment with ScTIL cells.

The liver function and HBV markers detected at baseline were normal, and the following items occurred after cell therapy:

1) ALT/AST was higher than the upper limit of normal range;
2) HBsAg was transient positive, HBsAb and/or other antibodies subsequently turned positive, while HBV DNA copy number and HBcAb IgM remained negative all the time;
3) The subjects had no subjective clinical features of acute hepatitis (poor appetite, jaundice, hepatomegaly and distending pain of hepatic region, etc.); and
4) The cell product used for reinfusion was negative for HBV test, and the plasma immunoglobulin maintained at a normal physiological level.

In order to determine the causes of the above phenomena, a number of experiments were designed and conducted, and the causes of these phenomena were analyzed by answering the following questions.

1) Was there HBV contamination of cell products or exogenous HBV infection?
Whether the cell products contained HBV and whether there was HBV in the peripheral blood of the subjects were tested, and the results were negative. Therefore, it can be considered that the cell products were not contaminated by HBV, and the subjects had no exogenous HBV infection during clinical trials.
2) Was it due to the decrease of immunoglobulin secondary to B cell reduction, which leads to increased susceptibility to HBV virus and secondary infection?
After the patients received ScTIL cell therapy, the peripheral blood lymphocyte count was observed to be lower than the baseline level before treatment; however, the plasma immunoglobulin concentration remained in the normal physiological range all the time. Therefore, this possibility can be ruled out.
3) Was abnormal liver function due to adverse reactions related to cell therapy or autoimmune liver injury?
ScTILs had no chemical toxicity, and its molecular mechanism of action can ensure that it can not damage autologous healthy tissues. Therefore, this possibility can be ruled out.

2. Basic information of patients

Case-1 (WJ)

Female, 36 years old, gastric signet-ring cell carcinoma with cervix uterus metastasis for 2 years. Sequencing test results: TMB1.03, neoantigen 16, HLA all heterozygous. On November 15, 2019, apheresis was performed to isolate mononuclear cells, and the total number of cells collected was 5.5 x10E9/62 ml. The preparation of ScTIL cells used in this example was substantially the same as in Example 5, except that the enhanced receptor V1E was replaced by the enhanced receptor V2E (SEQ ID NO: 2), which was used together with CD19-CAR to transfect TIL cells to obtain ScTILs. Preparation of therapeutic cells: ScTIL cells: 70 ml×2 bags + 30 mL×1 bag, and the number of total effective cells: 2.5×10E9. The cells were reinfused two times, and the dates were December 13, 2019 (70 ml, the effective cell number being 1.03 ×10E9) and December 18, 2019 (70+30 ml, the effective cell number being 1.47 x10E9), respectively. The two reinfusion processes proceeded smoothly. In both cases, the body temperature increased 6 hours after the completion of reinfusion, the patient had self-reported fear of cold, headache and mild nausea, without vomiting and other special discomfort, and the body temperature decreased to a normal level after 3 days of fever.

Detection of hepatitis B related indices:

1) No abnormality was found in liver enzyme profile and five items of hepatitis B detected on 2019/11/18 (before treatment) and 2019/12/16 (the day of the first cell reinfusion);

2) The liver enzyme profile was reexamined as normal 21 days after the second reinfusion (2020/01/09);

3) 64 days after the second reinfusion (2020/02/20), the further reexamination revealed elevated liver enzyme profile: ALT of 65 U/L (normal range: 0-50 U/L) and AST of 61 U/L (normal range: 0-40 U/L), and the five items of hepatitis B were not examined at that time;

4) 115 days after the second reinfusion (2020/04/15), the reexamination revealed that the liver enzymology indices had dropped to the normal range, but the five items of hepatitis B revealed that the hepatitis B surface antibody was positive and the core antibody was positive (the reexamination on the next day showed negative), and the copy number result of the HBV DNA sent was negative. In order to rule out HBV contamination of cell products, the products were sent out to a third party on the same day for testing "apheresis samples for cell preparation" and "finished cell sample", and all the test results of hepatitis B virus were negative.

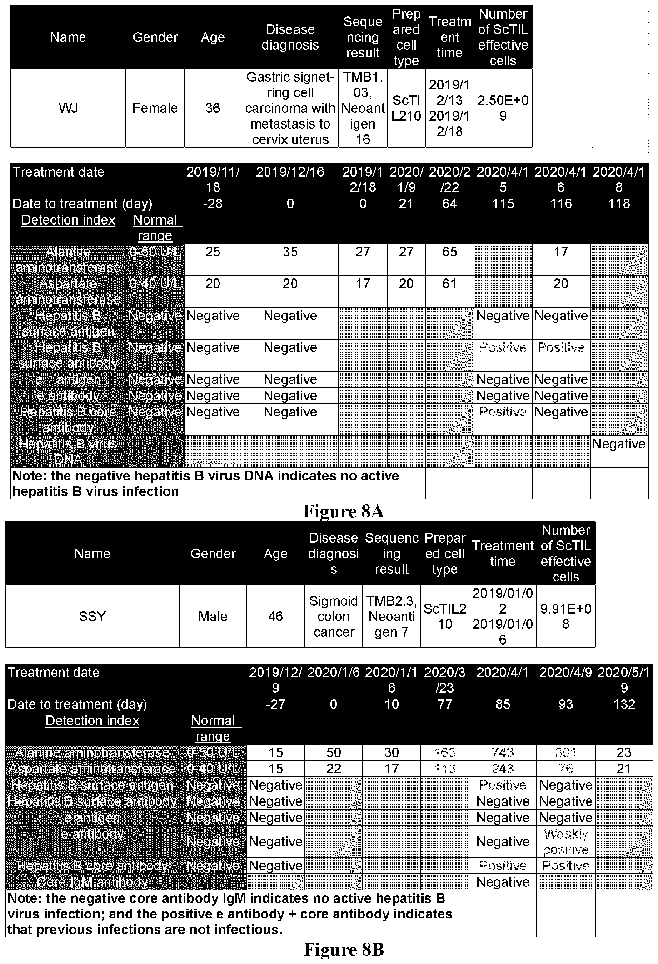

During the period when the above liver enzymes and five items of hepatitis B were abnormal, the patient had self-reported mild anorexia, but without nausea, vomiting, and distending pain and discomfort of hepatic region, and without common manifestations of acute infectious infections such as hepatomegaly and jaundice. The liver function- and HBV-related indices of the patient are shown in Figure 8A.

Case-2 (SSY)

Male, 46 years old, sigmoid colon cancer with peri-intestinal lymph node metastasis. Sequencing test results: TMB2.3, neoantigen 7, HLA all heterozygous. On November 15, 2019, apheresis was performed to isolate mononuclear cells, and the total number of collected cells was 4.66x10E9/54 ml (the current amount of remaining cells after preparation was 1.8 ×10E9); preparation of therapeutic cells: ScTIL cells: 50 ml×2 bags, with a total effective cell number of 9.91x10E8. The cells were reinfused two times, the dates were 2020/01/02 and 2020/01/06, respectively, the infusion number was 4.955x10E8/50ml for each of reinfusions, and the process proceeded smoothly. 7 hours after the first reinfusion, the fear of cold appeared and the body temperature increased up to 39.6°C. After oral administration of paracetamol plus physical cooling, the body temperature dropped to normal. The fever lasted for 3 days, and the patient felt no discomfort except fatigue, after which the body temperature dropped to a normal level. No obvious body temperature change and discomfort was seen after the second reinfusion.

Detection information of hepatitis B related indices:

1) The liver enzyme profile of the patient was examined 3 times, on 2019/12/09 (before treatment), 2020/01/06 (the day of the second cell reinfusion), and 2020/01/16 (day 10 after second cell reinfusion), and no abnormalities was seen; the five items of hepatitis B were negative before treatment;

2) 77 days after the second reinfusion (2020/03/23), the reexamination revealed elevated liver enzyme profile: ALT of 163 U/L (normal range: 0-50 U/L) and AST of 113 U/L (normal range: 0-40 U/L), and the five items of hepatitis B were not examined at that time;

3) 85 days after the second reinfusion (2020/04/01), the reexamination revealed ALT of 743 U/L (normal range: 0-50 U/L) and AST of 243 U/L (normal range: 0-40 U/L), and the five items of hepatitis B showed positive hepatitis B surface antigen, positive core antibody and negative core antibody IgM;

4) 93 days after the second reinfusion (2020/04/09), the reexamination revealed ALT of 301 U/L (normal range: 0-50 U/L) and AST of 76 U/L (normal range: 0-40 U/L), and the five items of hepatitis B showed that the hepatitis B surface antigen turned negative and the core antibody was still positive;

5) 132 days after the second infusion (2020/04/09), the reexamination revealed that both ALT and AST reduced to the normal range, and the five items of hepatitis B were not examined.

During the period when the above liver enzymes and five items of hepatitis B were abnormal, the patient had self-reported mild anorexia, but without nausea, vomiting, and distending pain and discomfort of hepatic region, and also without common manifestations of acute infectious infections such as hepatomegaly and jaundice. The liver function- and HBV-related indices of the patient are shown in Figure 8B.

Control case-real HBV infection (CY)

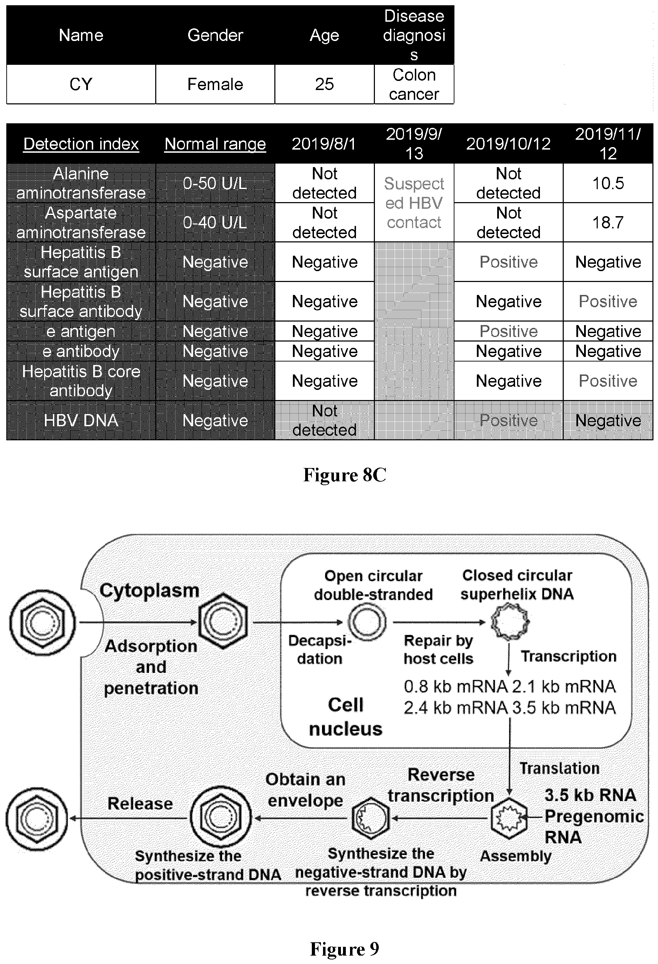

Female, 26 years old, with multiple lung metastases after colon cancer surgery, received ScTIL cell (the effective cell number being 6.4x10E8) reinfusion therapy on May 9, 2019. The patient was tested for hepatitis B markers on August 1, 2019, and the result was negative. On September 13, 2019, there was a highly suspicious blood-borne HBV contact event. On October 12, 2019, the test found that the hepatitis B surface antigen and e antigen were positive, the antibody was negative, and the hepatitis B virus DNA copy number was positive. After administrating liver protection treatment, the reexamination was performed on November 12, 2019, and the results showed that both the hepatitis B antigen and the hepatitis B virus DNA copy number turned negative, while the hepatitis B surface antibody and the core antibody turned positive. The liver function- and HBV-related indices of the patient are shown in Figure 8C.

3. Correlation between hepatitis B marker and liver enzyme abnormalities and cell therapy

The above two patients were characterized in that, while the anti-tumor efficacy of immune cells was observed, liver enzymes (ALT, AST) were detected to increase 6-8 weeks after cell reinfusion, HBsAg (surface antigen) turned positive transiently (turned negative after one week), then HBsAb/HBcAb (surface antibody/core antibody) turned from negative to positive and liver enzymes decreased to normal levels. During this period, both the hepatitis B virus (HBV) DNA copy number and HBcAb IgM detection for the peripheral blood and cell products were negative, and plasma immunoglobulins (IgG, IgM, IgA) were kept within the normal ranges.

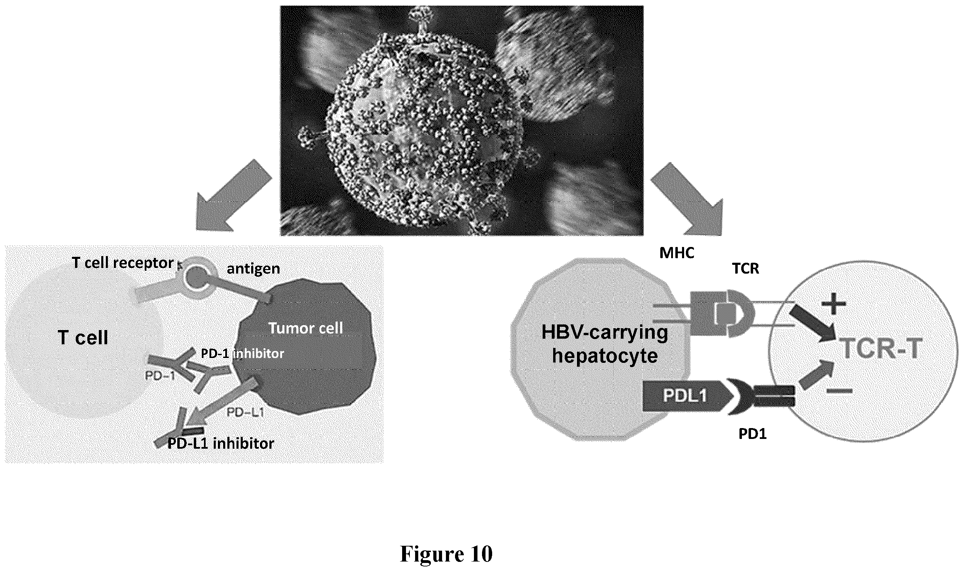

The inventor believes that this phenomenon is related to the fact that a small amount of viral components reside in liver cells after previous HBV infection (Figure 9), and the replication/assembly/exocytosis procedures of viral DNAs is silenced by the host's enhanced immune function. After HBV infection, the HBV protein presented by the MHC mechanism of liver cells can be recognized by immune lymphocytes; however, the attack of immune lymphocytes on target cells can be inhibited by the immune killing escape mechanism (PD-L1 of liver cells being expressed) of virus-infected liver cells, thereby preventing immunologic destruction of liver cells containing HBV components and HBV clearance (Figure 10), as a result, there is a risk of recurrence of hepatitis B infection when the body's immune function is significantly reduced.

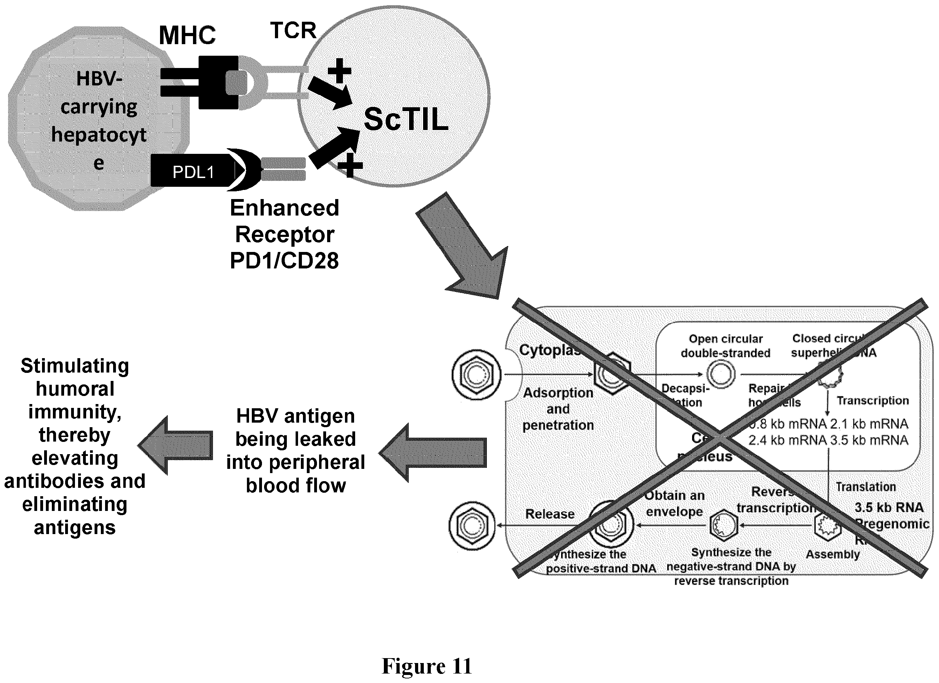

Mononuclear cells collected and used for preparation of ScTILs in the examples of the present application were prepared by sorting using PD-1 antibody magnetic beads and obtaining PD-1 positive (PD-1$^+$) lymphocytes. As mentioned hereinabove, these PD-1$^+$ cells contain CTL subsets that specifically recognize HBV-presenting membrane proteins, but whose killing activity on target cells is inhibited by PD-L1 expressed by liver cells. These cells were loaded with enhanced receptors by lentiviral transfection, thus reactivating their cell killing ability. After reinfusion, these modified cells (ScTILs) will recognize the HBV protein-expressing target cells via specific TCR, and the further activation of ScTIL and strengthened killing effect on the target cells will be triggered in turn by binding of the cell membrane enhanced factor, i.e. the extracellular domain of the PD-1 to PD-L1 highly expressed by the target cell reactivity. Liver cell disintegration by CTL attack will result in the release of HBV components into the peripheral blood flow, thereby stimulating humoral immunity and increasing antibody titers, which was manifested as transiently positive HBsAg and secondarily positive antibody (Figure 11). In the previous hepatitis B virus infection, the active virus particles that have been assembled in the host cell will not be affected by interferon inhibiting virus replication, and will not stay in the host cell, but will be released into the blood through the mechanism of exocytosis; therefore, when the liver cells disintegrate, the release of active HBV virus particles into the blood and the spread of infection will not happen. This hypothesis has been confirmed by negative test results for HBV DNA and HBcAb IgM.

In summary, based on the experimental results, the inventor inferred the following mechanism of action: ScTILs, as a combination of heterogeneous T lymphocyte subsets with specific antigen recognition ability, exerts an anti-malignant solid tumor effect, and at the same time, they can also incidentally eliminate the residual virus components hidden in some target cells after previous infections, thereby eliminating the potential risk of recurrence of previous viral infections in the future when immunity is low.

Therefore, the modified immune cells of the invention can eliminate viruses in the body and prevent recurrence of virus-related diseases while treating tumor diseases of tumor patients.

4. Use

Based on the above experimental findings, and the clinical significance of false HBV infection after ScTIL cell therapy, the following scenarios and use related to the ScTIL cell therapy of the invention are proposed.

4.1 Identification and diagnosis of false HBV infection and true HBV infection (see the control case) after cell therapy

Characteristics of false HBV infection after ScTIL cell therapy:

(1) HBV DNA copy number is below the positive threshold;
(2) Liver enzyme profile and HBV antigen abnormalities appear in early time and return to normal after 1-2 months; and
(3) HBV antibodies appear relatively late and maintain positive for a long time.

Characteristics of true HBV infection:

(1) The increase of HBV DNA copy number is accompanied by positive antigen and the titer is high;
(2) Liver enzyme abnormalities are usually positively correlated with quantitative abnormalities of HBV antigen.

4.2 Elimination of residual HBV virus components using ScTIL therapy

After the recovery from previous HBV infection, residual HBV components in the replication process in some liver cells may lead to recurrence of infection when the body's immunity is reduced.

Peripheral blood PD-1-positive lymphocytes comprise specific subsets, which can specifically recognize HBV membrane protein components of which the expression is presented to liver cell membranes, but can not kill target cells with residual virus components due to inhibition by secondary expression of PD-L1.

The enhanced receptor molecule of ScTIL can overcome the immune escape caused by PD-L1, eliminate the residual HBV virus components in the body, and eliminate the hidden danger of infection recurrence in the future.

**Example 7. Enhanced receptor-modified PD-1$^+$T cells to treat diseases related to HPV virus infection**

Using the method as described in Example 5, PD-1$^+$T cells were sorted based on peripheral blood samples from patients with HPV-positive tumors, and then ScTIL cells expressing enhanced receptors and optionally CD19 CAR were prepared. The prepared ScTIL cells were reinfused to the patients to treat tumors and eliminate HPV infection.

**Claims**

1. A modified immune cell, wherein the immune cell is a PD-1-positive T (PD-1$^+$T) cell derived from peripheral blood.

2. The modified immune cell of claim 1, wherein the PD-1$^+$T cell is derived from peripheral blood mononuclear cells (PBMCs).

3. The modified immune cell of claim 1 or 2, wherein the modification comprises a genetic engineering or a cell surface protein modification.

4. The modified immune cell of claim 3, wherein the modification results in loss or reduction of PD-1 expression, or inhibition of the function of PD-1 in the PD-1$^+$T cell.

5. The modified immune cell of claim 4, wherein the loss or reduction of PD-1 expression is performed by knocking out or knocking down a PD-1 gene.

6. The modified immune cell of claim 4, wherein the inhibition of the function of PD-1 is performed by contacting the immune cell with a PD-1 antibody *in vivo* or *in vitro,* the contacting causing the PD-1 antibody to bind to PD-1 on the surface of the immune cell.

7. The modified immune cell of any one of claims 1 to 6, wherein the modified immune cell comprises an enhanced receptor (ER) comprising an extracellular domain (ECD) and an intracellular domain (ICD), wherein the ECD is capable of binding to a target cell of the immune cell, and the ICD comprises a costimulatory molecule or a fragment thereof that elicits an immune cell activation signal.

8. The modified immune cell of claim 7, wherein the ECD comprises a receptor, a ligand and an antibody of a membrane protein of the target cell, or a portion or fragment of the receptor, the ligand and the antibody of the membrane protein of the target cell having the function of binding to the target cell.

9. The modified immune cell of claim 8, wherein the ECD comprises a partial sequence of PD1, or an anti-PD-L1 antibody, preferably an anti-PD-L1scFv; and/or the ICD is derived from CD28.

10. The modified immune cell of any one of claims 1 to 9, which has the ability of targeting to a target cell in a subject.

11. The modified immune cell of claim 10, wherein the target cell is selected from one or more of a group of: tumor cells, cancer cells, and virus-infected cells.

12. The modified immune cell of claim 11, wherein the virus is hepatitis virus, preferably hepatitis B virus, or human papilloma virus.

13. The modified immune cell of any one of claims 1 to 12, further expressing a chimeric antigen receptor (CAR), wherein the CAR specifically recognizes another antigen, preferably CD19, which is different from the antigen recognized by natural TCR of the immune cell.

14. The modified immune cell of any one of claims 1 to 13, further comprising other modifications that regulate the death of the immune cell, such as a suicide switch.

15. A cell population comprising the modified immune cell of any one of claims 1 to 14.

16. A method for preparing therapeutic immune cells, the method comprising:

(a) sorting PD-1$^+$T cells from peripheral blood; and
(b) performing one or more of the following treatments on the PD-1$^+$T cells sorted in step (a):

i. knocking out or knocking down the expression of PD-1;
ii. mixing with a PD-1 antibody;
iii. allowing the cell to express an enhanced receptor (ER);
iv. allowing the cell to express a chimeric antigen receptor (CAR); and
v. allowing the cell to express a suicide switch.

17. The modified immune cells obtained by the method of claim 19.

18. A composition comprising the immune cells of any one of claims 1-14 or 17 or the cell population of claim 15.

19. The composition of claim 18, for use in

(1) treatment of tumors, and/or
(2) treatment or prevention of diseases or symptoms related to viral infections, or prevention of recurrence of diseases or symptoms related to viral infections.

**Figure 1**

**Figure 2**

Figure 3

**A**

**B**

**Figure 4**

C

Figure 4 (continued)

**D**

**E**

**Figure 4 (continued)**

**A**

**B**

**Figure 5**

**C**

**D**

**Figure 5 (continued)**

**Figure 6**

**D**

**E**

**F**

**Figure 6 (continued)**

A

B

Figure 7

Figure 7 (continued)

| Name | Gender | Age | Disease diagnosis | Sequencing result | Prepared cell type | Treatment time | Number of ScTIL effective cells |
|---|---|---|---|---|---|---|---|
| WJ | Female | 36 | Gastric signet-ring cell carcinoma with metastasis to cervix uterus | TMB1.03, Neoantigen 16 | ScTIL210 | 2019/12/13 2019/12/18 | 2.50E+09 |

| Treatment date | | 2019/11/18 | 2019/12/16 | 2019/12/18 | 2020/1/9 | 2020/2/22 | 2020/4/15 | 2020/4/16 | 2020/4/18 |
|---|---|---|---|---|---|---|---|---|---|
| Date to treatment (day) | | -28 | 0 | 0 | 21 | 64 | 115 | 116 | 118 |
| Detection index | Normal range | | | | | | | | |
| Alanine aminotransferase | 0-50 U/L | 25 | 35 | 27 | 27 | 65 | | 17 | |
| Aspartate aminotransferase | 0-40 U/L | 20 | 20 | 17 | 20 | 61 | | 20 | |
| Hepatitis B surface antigen | Negative | Negative | Negative | | | | Negative | Negative | |
| Hepatitis B surface antibody | Negative | Negative | Negative | | | | Positive | Positive | |
| e antigen | Negative | Negative | Negative | | | | Negative | Negative | |
| e antibody | Negative | Negative | Negative | | | | Negative | Negative | |
| Hepatitis B core antibody | Negative | Negative | Negative | | | | Positive | Negative | |
| Hepatitis B virus DNA | | | | | | | | | Negative |

Note: the negative hepatitis B virus DNA indicates no active hepatitis B virus infection

**Figure 8A**

| Name | Gender | Age | Disease diagnosis | Sequencing result | Prepared cell type | Treatment time | Number of ScTIL effective cells |
|---|---|---|---|---|---|---|---|
| SSY | Male | 46 | Sigmoid colon cancer | TMB2.3, Neoantigen 7 | ScTIL210 | 2019/01/02 2019/01/06 | 9.91E+08 |

| Treatment date | | 2019/12/9 | 2020/1/6 | 2020/1/16 | 2020/3/23 | 2020/4/1 | 2020/4/9 | 2020/5/19 |
|---|---|---|---|---|---|---|---|---|
| Date to treatment (day) | | -27 | 0 | 10 | 77 | 85 | 93 | 132 |
| Detection index | Normal range | | | | | | | |
| Alanine aminotransferase | 0-50 U/L | 15 | 50 | 30 | 163 | 743 | 301 | 23 |
| Aspartate aminotransferase | 0-40 U/L | 15 | 22 | 17 | 113 | 243 | 76 | 21 |
| Hepatitis B surface antigen | Negative | Negative | | | | Positive | Negative | |
| Hepatitis B surface antibody | Negative | Negative | | | | Negative | Negative | |
| e antigen | Negative | Negative | | | | Negative | Negative | |
| e antibody | Negative | Negative | | | | Negative | Weakly positive | |
| Hepatitis B core antibody | Negative | Negative | | | | Positive | Positive | |
| Core IgM antibody | | | | | | Negative | | |

Note: the negative core antibody IgM indicates no active hepatitis B virus infection; and the positive e antibody + core antibody indicates that previous infections are not infectious.

**Figure 8B**

| Name | Gender | Age | Disease diagnosis |
|---|---|---|---|
| CY | Female | 25 | Colon cancer |

| Detection index | Normal range | 2019/8/1 | 2019/9/13 | 2019/10/12 | 2019/11/12 |
|---|---|---|---|---|---|
| Alanine aminotransferase | 0-50 U/L | Not detected | Suspected HBV contact | Not detected | 10.5 |
| Aspartate aminotransferase | 0-40 U/L | Not detected | | Not detected | 18.7 |
| Hepatitis B surface antigen | Negative | Negative | | Positive | Negative |
| Hepatitis B surface antibody | Negative | Negative | | Negative | Positive |
| e antigen | Negative | Negative | | Positive | Negative |
| e antibody | Negative | Negative | | Negative | Negative |
| Hepatitis B core antibody | Negative | Negative | | Negative | Positive |
| HBV DNA | Negative | Not detected | | Positive | Negative |

Figure 8C

Figure 9

39

Figure 10

Figure 11

**Figure 12**

**Figure 13**

**Figure 14**

**CD19 CAR**

**Figure 15**

Figure 16

## INTERNATIONAL SEARCH REPORT

| | International application No. |
|---|---|
| | **PCT/CN2021/126480** |

**A. CLASSIFICATION OF SUBJECT MATTER**

C12N 5/0783(2010.01)i; C12N 5/10(2006.01)i; C12N 15/12(2006.01)n; C12N 15/13(2006.01)n; A61P 31/12(2006.01)n; A61P 35/00(2006.01)n

According to International Patent Classification (IPC) or to both national classification and IPC

**B. FIELDS SEARCHED**

Minimum documentation searched (classification system followed by classification symbols)

C12N5/-; C12N15/-; A61P31/-; A61P35/-

Documentation searched other than minimum documentation to the extent that such documents are included in the fields searched

Electronic data base consulted during the international search (name of data base and, where practicable, search terms used)

CNABS, CNTXT, WPABS, WPABSC, ENTXT, ENTXTC, DWPA, CJFD, WBE OF SCIENCE: PD-1, PD1, 阳性, positive, +, T细胞, T淋巴细胞, T cell?, 肿瘤特异性T细胞, 肿瘤识别性T细胞, 耗竭性T细胞, 敲除, 敲低, knockout, knockdown

**C. DOCUMENTS CONSIDERED TO BE RELEVANT**

| Category* | Citation of document, with indication, where appropriate, of the relevant passages | Relevant to claim No. |
|---|---|---|
| PX | CN 112266899 A (BEIJING CHINEO MEDICAL TECHNOLOGY CO., LTD.) 26 January 2021 (2021-01-26) <br> claims 1-19 | 1-19 |
| X | CN 110016465 A (BEIJING CHINEO MEDICAL TECHNOLOGY CO., LTD.) 16 July 2019 (2019-07-16) <br> claims 1-3 | 1-3, 10, 11, 13-19 |
| Y | CN 110016465 A (BEIJING CHINEO MEDICAL TECHNOLOGY CO., LTD.) 16 July 2019 (2019-07-16) <br> claims 1-3 | 4-9, 12 |
| X | CN 110452870 A (HENAN CANCER HOSPITAL) 15 November 2019 (2019-11-15) <br> claims 1 and 3 | 1-4, 6, 10, 11, 15, 18, 19 |
| Y | 李卿 等 (LI, Qing et al.). "耗竭性T细胞在肿瘤中的作用 (The Role of Exhausted T Cells in Tumor)" <br> 现代生物医学进展 (Progress in Modern Biomedicine), <br> Vol. 17, No. 9, 31 March 2017 (2017-03-31), <br> ISSN: 1673-6273, <br> abstract, foreword, part 3 | 4-6, 12 |

☑ Further documents are listed in the continuation of Box C.    ☑ See patent family annex.

| *   Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **17 December 2021** | **06 January 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

# EP 4 257 676 A1

## INTERNATIONAL SEARCH REPORT

| | International application No. |
|---|---|
| | **PCT/CN2021/126480** |

### C. DOCUMENTS CONSIDERED TO BE RELEVANT

| Category* | Citation of document, with indication, where appropriate, of the relevant passages | Relevant to claim No. |
|---|---|---|
| Y | CN 111542595 A (BEIJING CHINEO MEDICAL TECHNOLOGY CO., LTD.) 14 August 2020 (2020-08-14) description paragraphs 55, 56 | 7-9 |
| A | CN 107249606 A (THE TRUSTEES OF THE UNIVERSITY OF PENNSYLVANIA) 13 October 2017 (2017-10-13) entire document | 1-19 |
| A | US 201615548202 A (JUNE CARL H et al.) 01 February 2016 (2016-02-01) entire document | 1-19 |
| A | 谢璐鸿 (XIE, Luhong). "免疫检查点PD-1及其抗体在肿瘤中的研究进 (non-official translation: Research Progress in Immune Checkpoint PD-1 and Antibodies for Same in Tumors" 检验医学与临床 (Laboratory Medicine and Clinic), Vol. 14, No. 9, 31 May 2017 (2017-05-31), ISSN: 1672-9455, pages 1353-1355, 1362 | 1-19 |
| A | Sunao Sugita et al. "T-Cell Suppression by Programmed Cell Death 1 Ligand 1 on Retinal Pigment Epithelium during Inflammatory Conditions" Investigative Ophthalmology & Visual Science, Vol. 50, No. 6, 30 June 2009 (2009-06-30), ISSN: 0146-0404, pp. 2862-2870 | 1-19 |

Form PCT/ISA/210 (second sheet) (January 2015)

47

## INTERNATIONAL SEARCH REPORT

| International application No. |
|---|
| **PCT/CN2021/126480** |

**Box No. I        Nucleotide and/or amino acid sequence(s) (Continuation of item 1.c of the first sheet)**

1.   With regard to any nucleotide and/or amino acid sequence disclosed in the international application, the international search was carried out on the basis of a sequence listing:

   a.   ☑   forming part of the international application as filed:

   ☑   in the form of an Annex C/ST.25 text file.

   ☐   on paper or in the form of an image file.

   b.   ☐   furnished together with the international application under PCT Rule 13*ter*.1(a) for the purposes of international search only in the form of an Annex C/ST.25 text file.

   c.   ☐   furnished subsequent to the international filing date for the purposes of international search only:

   ☐   in the form of an Annex C/ST.25 text file (Rule 13*ter*.1(a)).

   ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.   Additional comments:

Form PCT/ISA/210 (continuation of first sheet) (January 2015)

## INTERNATIONAL SEARCH REPORT
### Information on patent family members

International application No.

**PCT/CN2021/126480**

| Patent document cited in search report | | | Publication date (day/month/year) | Patent family member(s) | | | Publication date (day/month/year) |
|---|---|---|---|---|---|---|---|
| US | 201615548202 | A | 01 February 2016 | US | 11161907 | B2 | 02 November 2021 |
| | | | | WO | 2016126608 | A1 | 11 August 2016 |
| CN | 111542595 | A | 14 August 2020 | CN | 110042126 | A | 23 July 2019 |
| | | | | CN | 110016465 | A | 16 July 2019 |
| | | | | WO | 2019091478 | A1 | 16 May 2019 |
| | | | | EP | 3707248 | A1 | 16 September 2020 |
| | | | | EP | 3707248 | A4 | 18 August 2021 |
| | | | | JP | 2021502114 | W | 28 January 2021 |
| | | | | US | 2020354676 | A1 | 12 November 2020 |
| CN | 107249606 | A | 13 October 2017 | CA | 2964948 | A1 | 06 May 2016 |
| | | | | WO | 2016069282 | A1 | 06 May 2016 |
| | | | | AU | 2015339743 | A1 | 27 April 2017 |
| | | | | KR | 20170074245 | A | 29 June 2017 |
| | | | | EP | 3215168 | A1 | 13 September 2017 |
| | | | | US | 2017290858 | A1 | 12 October 2017 |
| | | | | JP | 2018500006 | W | 11 January 2018 |
| | | | | HK | 1243332 | A0 | 13 July 2018 |
| | | | | EP | 3215168 | A4 | 15 August 2018 |
| | | | | US | 2018312848 | A1 | 01 November 2018 |
| | | | | AU | 2015339743 | B2 | 05 November 2020 |
| | | | | AU | 2021200118 | A1 | 25 March 2021 |
| | | | | JP | 2021058196 | A | 15 April 2021 |
| | | | | AU | 2015339743 | C1 | 22 April 2021 |
| | | | | AU | 2021203981 | A1 | 15 July 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2020073017 W